# EUROPÄISCHE PATENTSCHRIFT

(11) **EP 2 432 820 B1**
(45) Veröffentlichungstag und Bekanntmachung des Hinweises auf die Patenterteilung: **24.10.2012**
(21) Anmeldenummer: 10719266.8
(22) Anmeldetag: 03.05.2010
(51) Int. Cl.: C08G 65/00

(54) **TERMINAL UNGESÄTTIGTE, OXETAN-BASIERTE MAKROMONOMERE UND VERFAHREN ZU DEREN HERSTELLUNG**
TERMINALLY UNSATURATED, OXETANE-BASED MACROMONOMERS, AND METHOD FOR THE PRODUCTION THEREOF
MACROMONOMÈRES À BASE D'OXÉTANE À INSATURATION TERMINALE ET LEUR PROCÉDÉ DE PRODUCTION

(30) Priorität: 19.05.2009 DE 102009021913
(43) Veröffentlichungstag der Anmeldung: 28.03.2012
(73) Patentinhaber: BYK-Chemie GmbH, 46483 Wesel (DE)
(72) Erfinder: FRANK, Albert, 46509 Xanten (DE); JAUNKY, Wojciech, 46485 Wesel (DE); ISSBERNER, Jörg, 47877 Willich (DE)
(74) Vertreter: Leifert & Steffan
(86) Internationale Anmeldenummer: PCT/EP2010/002672
(87) Internationale Veröffentlichungsnummer: WO 2010/133289

(56) Entgegenhaltungen:
- WO-A1-00/56802
- WO-A1-01/00701
- WO-A1-02/40572
- WO-A1-03/062306
- WO-A2-02/22700
- BEDNAREK M ET AL: "BRANCHED POLYETHER WITH MULTIPLE PRIMARY HYDROXYL GROUPS: POLYMERIZATION OF 3-ETHYL-3-HYDROXYMETHYLOXETANE", MACROMOLECULAR: RAPID COMMUNICATIONS, WILEY VCH VERLAG, WEINHEIM, DE, Bd. 20, Nr. 7, 1. Juli 1999 (1999-07-01), Seiten 369-372, XP000866715, ISSN: 1022-1336, DOI: DOI:10.1002/(SICI)1521-3927(19990701)20:7< 369::AID-MARC369>3.3.CO;2-J

## Beschreibung

Die Erfindung betrifft terminal ungesättigte, oxetan-basierte Makromonomere und ein Verfahren zu ihrer Herstellung.

### Stand der Technik

Makromonomere sind Oligomere oder Polymere mit mindestens einer funktionellen Endgruppe, über die Polymerisationsreaktionen ablaufen können. Makromonomere sind somit makromolekulare Monomere, die zu Homo- oder Copolymeren definierter Strukturen umgesetzt werden können. Beispielsweise können Makromonomere mit einer ethylenisch ungesättigten Endgruppe durch radikalische Polymerisation zu Propfcopolymeren oder Kammpolymeren mit definierter Länge der Seitenketten umgesetzt werden.

WO 02/40572 A1 beschreibt ein Verfahren zur Herstellung dendritischer Polyether mit einem Kern, welcher aus einer Verbindung mit zwei oder mehr Hydroxylgruppen stammt, und mindestens einer verzweigenden Generation, welche aus mindestens einem Hydroxyoxetan erhältlich ist. Der Kern des dendritischen Polyethers kann eine oder mehrere Allylgruppen enthalten. Allylgruppen weisen jedoch nur eine vergleichsweise geringe Reaktivität in radikalischen Polymerisationsreaktionen auf.

WO 00/56802 A1 beschreibt die Synthese hyperverzweigter dendritischer Polyether, die erhältlich sind durch thermisch initiierte kationische Ringöffnung von Oxetan-Monomeren mit mindestens zwei funktionellen Gruppen. Die terminalen Hydroxygruppen der erhaltenen hyperverzweigten dendritischen Polyether können dann weiter umgesetzt werden, beispielsweise durch Funktionalisierung mit Epoxid-, Allyl- oder Acrylatgruppen. Nach dieser Methode können jedoch keine definierten Polyether erhalten werden, die jeweils nur eine radikalisch polymerisierbare Gruppe pro Molekül enthalten.

Die radikalische Polymerisation solcher Makromonomeren, die mehrere radikalisch polymerisierbare Gruppen pro Molekül enthalten, beinhaltet die Gefahr unerwünschter Vernetzungsreaktionen.

### Aufgabe

Aufgabe der vorliegenden Erfindung war es, neue oxetan-basierte Makromonomere und ein Verfahren zu ihrer Herstellung bereitzustellen, die pro Molekül jeweils eine leicht radikalisch polymerisierbare Kopfgruppe enthalten und aus denen durch radikalische Homo- oder Copolymerisation, gegebenenfalls mit weiteren Comonomeren, neue Homo- und/oder Copolymere erhalten werden können, die als Additive in Beschichtungsmitteln und/oder Kunststoffen eingesetzt werden können.

Durch die Verwendung der neuen Homo- und/oder Copolymere als Additive sollen die Eigenschaften der Beschichtungsmittel bzw. Beschichtungen und/oder Kunststoffe gezielt verändert werden können.
- Beispielsweise sollen Additive erhalten werden können, die als Verlaufmittel geeignet sind und beispielsweise den Verlauf, den Glanz und/oder die Opaleszenz der Beschichtungsmittel bzw. Beschichtungen oder Kunststoffe verbessern können.
- Beispielsweise sollen Additive erhalten werden können, die den Beschichtungsmitteln bzw. Beschichtungen und/oder den Kunststoffen eine anti-adhäsive Oberfläche verleihen, so dass sich beispielsweise schmutz-, wasser- und/oder ölabweisende Oberflächen erzielen lassen.
- Beispielsweise sollen Additive erhalten werden können, die als Netz- und Dispergiermittel geeignet sind und beispielsweise die Viskosität von pigmentierten und/oder füllstoffhaltigen Beschichtungsmitteln erniedrigen und/oder die Dispersion von Pigmenten und/oder Füllstoffen in Beschichtungsmitteln stabilisieren können.
- Beispielsweise sollen Additive erhalten werden können, die den Beschichtungsmitteln bzw. Beschichtungen und/oder den Kunststoffen eine verbesserte Benetzbarkeit verleihen, so dass beispielsweise leichter bedruckbare Oberflächen erzielt werden und/oder die Haftung von Beschichtungen verbessert wird. Viele Beschichtungen, polymere Formmassen und Thermoplasten besitzen eine unpolare Oberfläche mit niedriger Oberflächenenergie. Deshalb ist die Benetzung derartiger Oberflächen durch z. B. wäßrige Beschichtungsmittel, Druckfarben, wässrige Kunststoffdispersionen, Klebstoffe oder Haftvermittler eine Herausforderung. Um die Benetzbarkeit derartiger Substratoberflächen durch polare (z.B. wäßrige) Beschichtungsmittel zu verbessern, muss die Oberflächenenergie der Substrate durch den Einsatz von Additiven erhöht werden, so dass die Substratoberflächen polarer gemacht werden. Aus diesem Grund gibt es einen Bedarf zur Entwicklung entsprechender Additive zur Verbesserung der Benetzbarkeit und damit z.B. der Bedruckbarkeit oder Lackierbarkeit diverser Beschichtungen, polymerer Formmassen und Thermoplasten.

Weiterhin sollen die zur Vermittlung dieser verbesserten Eigenschaften zugesetzten Additive die anderen Eigenschaften der Beschichtungsmittel bzw. Beschichtungen, polymeren Formmassen oder Thermoplasten möglichst nicht beeinträchtigen. Die zugesetzten Additive sollen außerdem in relativ geringen Mengen ihre Wirksamkeit entfalten können. Die Beschichtungen, polymeren Formmassen und Thermoplasten sollen ihre verbesserten Oberflächeneigenschaften weiterhin über einen langen Zeitraum von mehreren Jahren auch bei Außenbewitterung nahezu beibehalten. Dies sollte auch die Permanenz der Effekte, beispielsweise des anti-adhäsiven und/oder schmutzabweisenden Effektes, über mehrere Reinigungszyklen hinweg mit einschließen.

### Lösung

Diese Aufgaben werden überraschenderweise durch den Gegenstand der unabhängigen Patentansprüche gelöst. Besondere Ausführungsformen der Erfindung werden in den abhängigen Patentansprüchen beschrieben.

Ein Gegenstand der Erfindung ist ein Verfahren zur Herstellung eines Makromonomers, wobei mindestens ein Hydroxyoxetan der Formel (I) 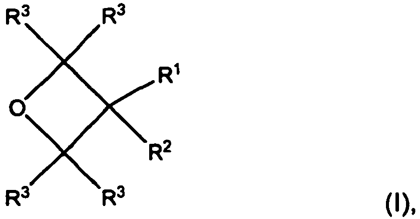 worin
R¹ für Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
R² für Hydroxyalkyl, Hydroxyalkoxy, Hydroxyalkoxyalkyl, Hydroxyaryl oder Hydroxyaryloxy steht, und
R³ unabhängig voneinander jeweils für Wasserstoff, Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
mit einem Startmolekül U - L - X - H, worin
U = eine von Allyl [H₂C = CH - CH₂ -] verschiedene, ethylenisch ungesättigte, radikalisch polymerisierbare Kopfgruppe,
L = zweibindiger organischer Rest ohne radikalisch polymerisierbare Gruppen, und
X -H = nicht radikalisch polymerisierbare Gruppe mit aktivem Wasserstoffatom H ist,
in einem molaren Verhältnis (Hydroxyoxetan(e) der Formel (I) : Startmolekül) von (100 : 1) bis (1 : 1) in Anwesenheit mindestens eines geeigneten Katalysators in einer kationischen ringöffnenden Polymerisation umgesetzt wird.

Ein weiterer Gegenstand der Erfindung sind Makromonomere, die nach diesem Verfahren erhältlich sind.

### Ausführliche Beschreibung der Erfindung

Es ist erfindungswesentlich, dass die durch das erfindungsgemäße Verfahren erhältlichen, erfindungsgemäßen oxetan-basierten Makromonomere pro Molekül jeweils eine radikalisch polymerisierbare Kopfgruppe enthalten. Hierdurch wird es ermöglicht, dass bei einer anschließenden radikalischen Homo- oder Copolymerisation Polymere mit definierter Kammstruktur erhalten werden. Es ist des weiteren erfindungswesentlich, dass die eine Kopfgruppe leicht radikalisch polymerisierbar ist. Daher scheiden Allylgruppen [H₂C = CH - CH₂ -], die nur eine geringe Tendenz zur radikalischen Polymerisation aufweisen, als Kopfgruppen aus.

### Hydroxyoxetane

In dem erfindungsgemäßen Verfahren wird mindestens ein Hydroxyoxetan der Formel (I) 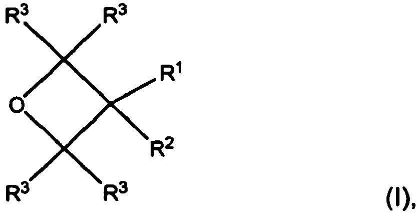 worin
R¹ für Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
R² für Hydroxyalkyl, Hydroxyalkoxy, Hydroxyalkoxyalkyl, Hydroxyaryl oder Hydroxyaryloxy steht, und
R³ unabhängig voneinander jeweils für Wasserstoff, Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
mit einem Startmolekül wie oben beschrieben umgesetzt.

Bevorzugt ist das mindestens eine Hydroxyoxetan der Formel (I)
ein 3-Alkyl-3-(hydroxyalkyl)oxetan [d.h. R¹ = Alkyl, R² = Hydroxyalkyl, alle R³ = H],
ein 3,3-Di(hydroxyalkyl)oxetan [d.h. R¹, R² = Hydroxyalkyl, alle R³ = H],
ein 3-Alkyl-3-(hydroxyalkoxy)oxetan [d.h. R¹ = Alkyl, R² = Hydroxyalkoxy, alle R³ = H],
ein 3-Alkyl-3-(hydroxyalkoxyalkyl)oxetan [d.h. R¹ = Alkyl, R² = Hydroxyalkoxyalkyl, alle R³ = H],
oder ein Gemisch dieser Hydroxyoxetane,
besonders bevorzugt ein 3-Alkyl-3-(hydroxyalkyl)oxetan [d.h. R¹ = Alkyl, R² = Hydroxyalkyl, alle R³ = H] und/oder ein 3-Alkyl-3-(hydroxyalkoxy)oxetan [d.h. R¹ = Alkyl, R² = Hydroxyalkoxy, alle R³ = H].

Der Ausdruck "Alkyl" steht hier vorzugsweise für lineare oder verzweigte Alkylreste mit 1 bis 24 C-Atomen, beispielsweise mit 1 bis 12 oder mit 1 bis 8 C-Atomen. Besonders bevorzugt steht der Ausdruck "Alkyl" für Methyl oder Ethyl.

Der Ausdruck "Alkoxy" steht hier vorzugsweise für Methoxy, Ethoxy, Propoxy, Butoxy, Phenylethoxy, besonders bevorzugt Ethoxy und/oder Propoxy, und umfasst bis zu 20 Alkoxyeinheiten oder eine Kombination von zwei oder mehr AlkoxyEinheiten, die statistisch, blockweise oder gradientenartig angeordnet sein können.

Beispiele für besonders geeignete Hydroxyoxetane sind
3-Methyl-3-(hydroxymethyl)oxetan [d.h. R¹ = Methyl, R² = Hydroxymethyl, alle R³ = H],
3-Ethyl-3-(hydroxymethyl)oxetan [d.h. R¹ = Ethyl, R² = Hydroxymethyl, alle R³ = H] (= Trimethylolpropanoxetan) sowie dessen an der Hydroxygruppe ethoxylierte und/oder propoxylierte Derivate mit 1-20 Ethylenoxid- und/oder Propylenoxid-Einheiten, die statistisch, blockweise oder gradientenartig angeordnet sein können,
3,3-Di(hydroxymethyl)oxetan [d.h. R¹ = R² = Hydroxymethyl, alle R³ = H],
oder ein Gemisch dieser Hydroxyoxetane.

Besonders bevorzugt werden 3-Ethyl-3-(hydroxymethyl)oxetan (= Trimethylolpropanoxetan, TMPO) und/oder dessen an der Hydroxygruppe ethoxylierte und/oder propoxylierte Derivate mit 1-20 Ethylenoxid- und/oder Propylenoxid-Einheiten, die statistisch, blockweise oder gradientenartig angeordnet sein können, eingesetzt.

Ein Vorteil der auf Basis von ethoxyliertem TMPO hergestellten Makromonomere im Vergleich zu linearen Polyethylenglykolmethacrylaten ist, dass sie bei gleichem Molgewicht einen niedrigeren Schmelzpunkt (eine geringere Kristallinität) und eine geringere Viskosität aufweisen. Diese Eigenschaften finden sich auch bei den aus diesen Makromonomeren hergestellten Polymeren wieder.

Die Hydroxygruppe oder die Hydroxygruppen des Hydroxyoxetans können frei vorliegen oder durch geeignete Schutzgruppen geschützt sein. Unter Schutzgruppen werden leicht spaltbare Gruppen wie beispielsweise Acetale verstanden.

Die Hydroxygruppe oder die Hydroxygruppen des Hydroxyoxetans können auch für Derivatisierungen genutzt werden. Beispielsweise können die Hydroxygruppen des Hydroxyoxetans mit Ethylenoxid, Propylenoxid, Butylenoxid oder Styroloxid alkoxyliert werden. Es können auch Mischungen der Alkylenoxide eingesetzt werden. Bevorzugt werden die Hydroxyoxetane vor der ringöffnenden Polymerisation ethoxyliert und/oder propoxyliert. Weiterhin können beispielsweise weitere Seitenketten durch die Umsetzung der Hydroxylgruppen mit Isocyaten unter Bildung von Urethanen eingeführt werden. So können z.B. Polyethylenoxid (EO)-, Polypropylenoxid (PO)- oder gemischte EO-PO-Seitenketten (hierin EO/PO als statistisches Polymer, Block- oder Gradientenpolymer) als gepropfte Seitenketten dadurch eingeführt werden, dass die entsprechenden Monoaddukte des oder der Polyalkylenoxide mit TDI (Toluylendiisocyanat) mit den Hydroxylgruppen des Hydroxyoxetans umgesetzt werden. Weitere mögliche Derivatisierungen der Hydroxylgruppen des Hydroxyoxetans sind z.B. Williamson sche Ethersynthese, Reaktionen mit Säureanhydriden oder Carboxymethylierung. Prinzipiell sind alle bekannten Derivatisierungen möglich, solange diese keinen negativen Einfluss auf die spätere kationische Ringöffnungspolymerisation der Hydroxyoxetane oder auf die bei der Verwendung der entstandenen oxetan-basierten Makromonomere stattfindende radikalische Polymerisation der ungesättigten Kopfgruppen der entstandenen oxetan-basierten Makromonomere haben. Durch eine Derivatisierung der Hydroxyoxetane können die Eigenschaften der erfindungsgemäßen oxetan-basierten Makromonomere beeinflußt werden, so dass bei der Verwendung der erfindungsgemäßen Makromonomere zur Herstellung von Polymeren auch die Eigenschaften der Polymere, beispielsweise hinsichtlich der Verträglichkeit mit verschiedenen Beschichtungsmitteln und Kunststoffen, beeinflusst werden können.

### Startmoleküle U - L - X - H

Das Startmolekül U - L - X - H enthält eine von Allyl [H₂C = CH - CH₂ -] verschiedene, ethylenisch ungesättigte, radikalisch polymerisierbare Kopfgruppe U. Es ist erfindungswesentlich, dass die eine Kopfgruppe leicht radikalisch polymerisierbar ist. Daher scheiden Allylgruppen [H₂C = CH - CH₂ -], die nur eine geringe Tendenz zur radikalischen Polymerisation aufweisen, als Kopfgruppen aus. Bevorzugt ist die ethylenisch ungesättigte Kopfgruppe U = Acryloyl [H₂C = CH - C(=O)-] oder Methacryloyl [H₂C = C(CH₃) - C(=O)-].

Der zweibindige organische Rest L enthält keine radikalisch polymerisierbaren Gruppen. Unter radikalisch polymerisierbaren Gruppen werden funktionelle Gruppen verstanden, die unter hierfür üblichen Bedingungen radikalisch polymerisiert werden können, beispielsweise ethylenisch ungesättigte Gruppen. Der Rest L kann beispielsweise ein Alkylen-, Arylen- oder Polyalkylenoxidrest sein.

Bevorzugt weist der zweibindige organische Rest L die Struktur -O-L auf, wobei -O-ein Sauerstoffatom ist und L' aus der Gruppe bestehend aus Alkylenresten und Polyalkylenoxidresten ausgewählt wird.

Besonders bevorzugt weist der zweibindige organische Rest L die Struktur -O-L' auf, wobei -O- ein Sauerstoffatom ist und L' aus der Gruppe, bestehend aus Alkylenresten mit 2 - 5 Kohlenstoffatomen, Polyethylenoxid-Resten mit 2 - 5 Ethylenoxid-Einheiten, Polypropylenoxid-Resten mit 2 - 5 Propylenoxid-Einheiten und gemischten Polyethylen/Polypropylen-Resten mit 2 - 5 Alkylenoxid-Einheiten, ausgewählt wird.

Ganz besonders bevorzugt weist der zweibindige organische Rest L die Struktur -O-L' auf, wobei -O- ein Sauerstoffatom ist und L' ein Alkylenrest mit 2 - 5 Kohlenstoffatomen ist.

Die Gruppe X - H ist eine nicht radikalisch polymerisierbare funktionelle Gruppe mit aktivem Wasserstoffatom H. Unter einem aktiven Wasserstoffatom wird ein Wasserstoffatom verstanden, das unter saurer Katalyse, beispielsweise mit einer Lewis-Säure (z.B. AlCl₃, BF₃, TiCl₄, Znl₂, SiF₄, SbF₅, PF₅, AsF₅ oder SbCl₅) oder beispielsweise mit einer halogenierten Säure (z.B. FSO₃H, CISO₃H, HClO₄, HIO₄ oder CF₃SO₃H), oder einer sonstigen Brönsted-Säure (z.B. Paratoluolsulfonsäure, Sulfonsäure) bei 30°C bis 130°C die kationische ringöffnende Polymerisation von Oxetanen initiieren kann. Die Gruppe X - H kann beispielsweise eine Gruppe N - H, eine Gruppe S - H oder eine Gruppe O - H sein. Bevorzugt ist die Gruppe X - H eine Hydroxygruppe O - H oder eine Thiolgruppe S - H, da hierdurch in der Regel höhere Ausbeuten erzielt werden können. Besonders bevorzugt ist die Gruppe X - H eine Hydroxygruppe O - H.

Das Startmolekül U - L - X - H wird vorzugsweise aus der Gruppe bestehend aus Hydroxyalkyl-Estern der Methacrylsäure [H₂C = C(CH₃) - C(=O) - O - (CH₂)ₐ - OH] und Polyethylenoxid-Estem der Methacrylsäure [H₂C = C(CH₃) - C(=O) - O - (CH₂ - CH₂ - O)ₐ - H] mit a = 1 - 20, bevorzugt a = 1 - 10 und besonders bevorzugt a = 2 - 5, ausgewählt.

Ganz besonders bevorzugt ist das Startmolekül U - L - X - H Hydroxyethylmethacrylat oder Hydroxybutylmethacrylat.

### Herstellung der oxetan-basierten Makromonomere

Die erfindungsgemäßen oxetan-basierten Makromonomere sind durch das erfindungsgemäße Herstellverfahren erhältlich, bei dem mindestens ein Hydroxyoxetan der Formel (I) 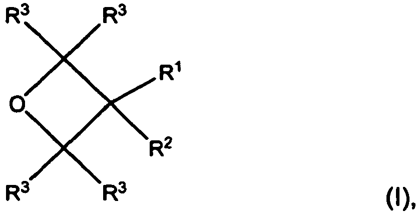 worin
R¹ für Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
R² für Hydroxyalkyl, Hydroxyalkoxy, Hydroxyalkoxyalkyl, Hydroxyaryl oder Hydroxyaryloxy steht, und
R³ unabhängig voneinander jeweils für Wasserstoff, Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
mit einem Startmolekül U - L - X - H, worin
U = eine von Allyl [H₂C = CH - CH₂ -] verschiedene, ethylenisch ungesättigte, radikalisch polymerisierbare Kopfgruppe,
L = zweibindiger organischer Rest ohne radikalisch polymerisierbare Gruppen, und X -H = nicht radikalisch polymerisierbare Gruppe mit aktivem Wasserstoffatom H ist,
in einem molaren Verhältnis (Hydroxyoxetan(e) der Formel (I) : Startmolekül) von (100 : 1) bis (1 : 1) in Anwesenheit mindestens eines geeigneten Katalysators in einer kationischen ringöffnenden Polymerisation umgesetzt wird.

Bevorzugt beträgt das molare Verhältnis (Hydroxyoxetan(e) der Formel (I) : Startmolekül) von (50 : 1) bis (2 : 1), besonders bevorzugt von (25 : 1) bis (3:1).

Es ist erfindungswesentlich, dass die erfindungsgemäßen oxetan-basierten Makromonomere pro Molekül jeweils eine radikalisch polymerisierbare Gruppe als Kopfgruppe enthalten. Hierdurch wird es ermöglicht, dass bei einer anschließenden radikalischen Homo- oder Copolymerisation der Makromonomere Polymere mit definierter Kammstruktur erhalten werden. Bekannterweise fallen jedoch bei chemischen Synthesen regelmäßig Nebenprodukte an. Bei der Synthese der erfindungsgemäßen Makromonomere können daher auch geringe Anteile von Makromonomeren mit zwei oder mehr radikalisch polymerisierbaren Gruppen sowie geringe Anteile von Produkten ohne eine radikalisch polymerisierbare Gruppe anfallen. Durch das erfindungsgemäße Herstellverfahren können die Anteile solcher Nebenprodukte jedoch sehr gering gehalten werden. Bevorzugt weisen die erfindungsgemäß synthetisierten Makromonomere daher nur sehr geringe, besonders bevorzugt keine Anteile von Molekülen mit keiner oder mehr als einer radikalisch polymerisierbaren Gruppe auf.

Die Herstellung der efindungsgemäßen Makromonomere durch kationische oxetanringöffnende Polymerisation der Hydroxyoxetane mit dem Startmolekül erfolgt bei etwa 30°C bis 130°C, vorzugsweise bei etwa 50°C bis 110°C, in Anwesenheit mindestens eines geeigneten Katalysators.

Als Katalysatoren für die kationische oxetanringöffnende Polymerisation sind beispielsweise Lewis-Säuren, wie insbesondere AlCl₃, BF₃, TiCl₄, Znl₂, SiF₄, SbF₅, PF₅, AsF₅ oder SbCl₅, sowie beispielsweise halogenierte Säuren, wie insbesondere FSO₃H, ClSO₃H, HClO₄, HIO₄ oder CF₃SO₃H, sowie weitere Brönsted-Säuren (z.B. Paratoluolsulfonsäure, Sulfonsäure) geeignet. Als Katalysatoren können auch Oniumsalze, wie beispielsweise ein Sulphonium-, ein Oxonium- und/oder lodoniumsalz, eingesetzt werden. Beispiele für derartige Verbindungen sind Benzyltetramethylensulphonium-hexafluoroantimonat, Benzyltetramethylensulphoniumhexafluorophosphat und Benzyltetramethylensulphonium-trifluormethansulphonat. Bevorzugt wird als Katalysator CF₃SO₃H (Trifluormethansulfonsäure) eingesetzt, da die ethylenisch ungesättigten Kopfgruppen des Startmoleküls U - L - X - H während der kationischen oxetanringöffnenden Polymerisation möglichst nicht beeinträchtigt, insbesondere nicht vorzeitig polymerisiert werden sollen, sondern die ungesättigten Gruppen möglichst vollständig erhalten bleiben sollen. Dies wird durch die Verwendung eines in dieser Hinsicht schonenden Katalysators der kationischen Polymerisation, wie beispielsweise Trifluormethansulfonsäure, erleichtert.

Bevorzugt erfolgt die Herstellung der erfindungsgemäßen Makromonomere in Anwesenheit eines Inhibitors der radikalischen Polymerisation. Als Inhibitoren der radikalischen Polymerisation sind beispielsweise Hydrochinon oder Hydrochinonmonomethylether geeignet. Der Inhibitor der radikalischen Polymerisation wird eingesetzt, um freie Radikale abzufangen, die sich möglicherweise während der Lagerung oder bei der thermisch unterstützten kationischen ringöffnenden Polymerisation der Oxetan-Monomere bilden könnten, so dass die Stabilität der ethylenisch ungesättigten, radikalisch polymerisierbaren Kopfgruppe U des Startmoleküls gewährleistet ist. Ein Vorteil bei der Verwendung eines Inhibitors der radikalischen Polymerisation liegt somit darin, dass die Reaktionsführung erleichtert wird und weniger Nebenprodukte anfallen.

Bevorzugt werden das oder die Hydroxyoxetane langsam zu dem vorgewärmten Reaktionsgemisch gegeben, das das Startmolekül U - L - X - H und den mindestens einen sauren Katalysator, sowie bevorzugt auch den mindestens einen Inhibitor der radikalischen Polymerisation, enthält. Durch die langsame Zugabe des oder der Hydroxyoxetane können besser definierte Produkte erhalten werden.

Es können Lösemittel verwendet werden. Diese dürfen die kationische oxetanringöffnende Polymerisation jedoch nicht beeinflussen. Geeignete Lösemittel sind beispielsweise solche, die keine aktiven Wasserstoffatome, keine polymerisierbaren Ringe und keine Gruppen enthalten, die mit dem Hydroxyoxetan (insbesondere mit den Hydroxylgruppen des Hydroxyoxetans), mit dem Startmolekül oder mit dem entstehenden oxetan-basierten Makromonomer reagieren könnten. Vorzugsweise können aliphatische, cycloaliphatische und aromatische Lösemittel, Ketone und blockierte Polyether als Lösemittel eingesetzt werden. Die Wahl des Lösemittels richtet sich auch nach dem späteren Einsatzzweck eines im Anschluß aus den Makromonomeren zu synthetisierenden Polymeren. Bevorzugt werden niedrigsiedende Lösemittel eingesetzt, um das Abdestillieren bei solchen Anwendungen zu erleichtern, bei denen die später erhaltenen Polymere als 100%-Formulierung eingesetzt werden sollen, z.B. bei UV-härtenden Lacksystemen.

Am Ende der Reaktion wird das Produkt abgekühlt und durch Zugabe einer Base oder durch Behandlung mit einem basischen Ionenaustauscherharz neutralisiert. Eventuelle Restmengen an Startmolekül können, sofern sie bei der nachfolgenden radikalischen Polymerisation stören, durch geeignete destillative Maßnahmen, wie z.B. durch Dünnschichtverdampfung, entfernt werden.

Die Durchführung der kationischen ringöffnenden Polymerisation der Hydroxyoxetane mit dem Startmolekül U - L - X - H wird durch die Auswahl bestimmter Startmoleküle U - L - X - H erleichtert. Die Reaktionsführung gelingt in der Regel besonders gut mit Startmolekülen aus der Gruppe der Ester der (Meth)Acrylsäure. Dagegen kann die Reaktionsführung beim Einsatz entsprechender Amide oder von Vinylbenzylalkoholen als Startmoleküle etwas schwieriger sein.

Das oxetan-basierte Makromonomer kann vor einer später folgenden radikalischen Polymerisation noch weiter durch polymeranaloge Reaktionen modifiziert werden. Beispielsweise können freie Hydroxylgruppen ganz oder teilweise verestert werden, zum Beispiel mit Essigsäure. Eine Veresterung kann beispielsweise nützlich sein, um bei Einsatz der Polymere in Lacksystemen Zwischenschichthaftungsprobleme zu vermeiden oder um die Polarität des Polymers zu verändern. Freie Hydroxylgruppen des oder der Makromonomere können auch ganz oder teilweise mit Isocyanaten umgesetzt werden, beispielsweise mit Alkylisocyanaten. Durch eine Modifikation mit längerkettigen Alkylisocyanaten, wie z.B. Stearylisocyanat, vor der radikalischen Polymerisation kann die Polarität des Polymers so in Richtung Hydrophobie verändert werden, dass ein Einsatz der Polymere in unpolaren Thermoplasten erleichtert wird. Dagegen erzielt man eine Erhöhung der Hydrophilie und gegebenenfalls verbesserte Wasserlöslichkeit dadurch, dass freie Hydroxylgruppen des oder der Makromonomere vor der radikalischen Polymerisation ganz oder teilweise mit hydrophilen Verbindungen, beispielsweise mit TDI-Monoaddukten umgesetzt werden. Geeignet sind z.B. Monoaddukte, die aus der Umsetzung von Toluylendiisocyanat (TDI) mit methanolgestartetem Polyethylenglykol. Durch entsprechende Mischungen von hydrophilen und hydrophoben Modifizierungen der Makromonomere vor der radikalischen Polymerisation lassen sich auch amphiphile Polymerstrukturen herstellen.

### Verwendung

Aus den erfindungsgemäßen Makromonomeren und gegebenenfalls weiteren Comonomeren sind Polymere dadurch erhältlich, dass ein oder mehrere der Makromonomere, die durch Umsetzung mindestens eines Hydroxyoxetans der Formel (I) 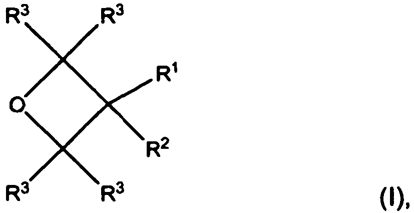 worin
R¹ für Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
R² für Hydroxyalkyl, Hydroxyalkoxy, Hydroxyalkoxyalkyl, Hydroxyaryl oder Hydroxyaryloxy steht, und
R³ unabhängig voneinander jeweils für Wasserstoff, Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
mit einem Startmolekül U - L - X - H, worin
U = eine von Allyl [H₂C = CH - CH₂ -] verschiedene, ethylenisch ungesättigte, radikalisch polymerisierbare Kopfgruppe,
L = zweibindiger organischer Rest ohne radikalisch polymerisierbare Gruppen, und X -H = nicht radikalisch polymerisierbare Gruppe mit aktivem Wasserstoffatom H ist,
in einem molaren Verhältnis (Hydroxyoxetan(e) der Formel (I) : Startmolekül) von (100 : 1) bis (1 : 1) in Anwesenheit mindestens eines geeigneten Katalysators in einer kationischen ringöffnenden Polymerisation erhältlich sind,
und gegebenenfalls ein oder mehrere weitere, radikalisch polymerisierbare Comonomere radikalisch polymerisiert werden.

Zur Herstellung der Polymere können ein oder mehrere verschiedene der erfindungsgemäßen Makromonomere radikalisch homo- oder copolymerisiert werden. Unter den erfindungsgemäßen Makromonomeren werden zur Herstellung der Polymere diejenigen Makromonomere bevorzugt eingesetzt, die nach den oben beschriebenen bevorzugten Ausführungsformen erhalten werden.

Zusätzlich zu dem oder den erfindungsgemäßen Makromonomeren können ein oder mehrere, davon verschiedene Comonomere eingesetzt werden, die radikalisch polymerisierbar sind. Vorzugsweise wird mindestens ein solches Comonomer eingesetzt. Unter radikalisch polymerisierbaren Comonomeren werden solche Verbindungen verstanden, die mindestens eine radikalisch polymerisierbare Gruppe tragen. Unter radikalisch polymerisierbaren Gruppen werden funktionelle Gruppen verstanden, die unter hierfür üblichen Bedingungen radikalisch polymerisiert werden können, beispielsweise ethylenisch ungesättigte Gruppen. Vorzugsweise wird diese ethylenisch ungesättigte Gruppe ausgewählt aus der Gruppe bestehend aus substituierten oder unsubstituierten Acryl-, Methacryl-, Acrylamido-, Methacrylamido-, Acrylamidoalkyl-, Methacrylamidoalkyl-, Styryl-, alpha-Methyl-Styryl-, Allyl-, Vinylbenzyl-, Vinylether-, Vinylester- und Vinylketongruppen; insbesondere, wenn die verwendeten Makromonomere als Kopfgruppe U Acryl-, Methacryl- oder Styrolgruppen tragen.

Beispiele geeigneter Comonomere sind Alkylacrylate und Alkylmethacrylate von geradkettigen, verzweigten oder cycloaliphatischen Alkoholen mit 1 bis 24 C-Atomen, Aralkylacrylate und Aralkylmethacrylate von Aralkylalkoholen mit 8 bis 18 C-Atomen, Hydroxyalkyl(meth)acrylate von geradkettigen, verzweigten oder cycloaliphatischen Diolen mit 2 bis 36 C-Atomen, Alkyl(meth)acrylamide von geradkettigen, verzweigten oder cycloaliphatischen Aminen mit 1 bis 22 C-Atomen, Aminoalkyl(meth)acrylate von geradkettigen, verzweigten oder cycloaliphatischen Aminoalkoholen mit 2 bis 8 C-Atomen, alkoxylierte (Meth)acrylate (wie beispielsweise Polyethylenoxid-(Meth)acrylate, Polypropylenoxid-(Meth)acrylate, Polyethylenoxid-Polypropylenoxid-(Meth)acrylate), polyestermodifizierte (Meth)acrylate (wie beispielsweise Caprolacton-modifizierte (Meth)acrylate, erhältlich z.B. von Daicel, Japan), (Meth)acrylnitrile, Vinylalkanoate, Alkene und Arylalkene mit 2 bis 30 C-Atomen, Styrole und substituierte Styrole, alpha-Methylstyrole und substituierte alpha-Methylstyrole, Alkylvinylether, Perfluoroalkyl(meth)acrylate sowie entsprechende teilweise fluorierte (Meth)acrylate, Perfluoroalkyl-Blockpolyether-(Meth)acrylate, Perfluoroalkylethylthiocarbonylaminoethyl-(Meth)acrylate, Perfluoroolefine, (Meth)acryloxyalkylsiloxane, (Meth)acryloxyalkylpolysiloxane, (Meth)acryloxypolyetheralkylpolysiloxane, N-Vinylcarbazole, fluorierte und fluor-freie Alkylester der Maleinsäure, der Fumarsäure, der Itaconsäure und der Mesaconsäure (Methylfumarsäure), (Meth)acrylamid, (Meth)acrylamide, Methacrylsäure, Acrylsäure, hydroxyfunktionelle Alkyl-(Meth)acrylamide, hydroxyfunktionelle Alkylvinylether, Natriumvinylsulfonat, Natriumstyrolsulfonat, 2-Acrylamido-2-methylpropansulfonsäure, N-Vinylpyrrol, N-Vinyl-2-pyrrolidon, 2-Vinyloxazolidin, 2-Vinylpyridin, 4-Vinylpyridin, 1-[2-(Methacrylolyoxy)-ethyl]-2-imidazolidinon, Aminoalkyl(meth)-acrylate, wie zum Beispiel N,N-Dimethylaminoethyl(meth)acrylat und N,N-Dimethylaminopropyl(meth)acrylat, N-Alkyl- und N,N-Dialkyl-substituierte Acrylamide mit geradkettigen, verzweigten oder cycloaliphatischen Alkylgruppen mit 1 bis 22 C-Atomen, wie zum Beispiel N-(t-Butyl)acrylamid und N,N-Dimethylacrylamid; ionische Monomere, Allylalkohol, alkoxylierte Allylalkohol-Derivate (wie beispielsweise ethoxylierter Allylalkohol, propoxylierter Allylalkohol, und mischalkoxylierter Allylalkohol mit Anteilen Ethylenoxid und Propylenoxid) sowie polyestermodifizierte (beispielsweise mit Caprolacton) Derivate auf Basis Allylalkohol.

Bevorzugt werden Comonomere wie Polydimethylsiloxanmono(meth)acrylate, beispielsweise lineare (Meth)acrylolxyalkylpolydimethylsiloxane (z.B. α-Butyldimethylsiloxy-ω-(3-methacryloxypropyl)-polydimethyisiloxan) oder α-Butyldimethyl-siloxy-ω-(3-acryloxy-2-hydroxypropoxypropyl)-polydimethylsiloxan), verzweigte (Meth)acryloxyalkylpolydimethylsiloxane (z.B. Methacryloxypropyl-terminierte, verzweigte Polydimethylsiloxane wie (Methacryloxypropyl)methylsiloxan-polydimethylsiloxan-Copolymere oder Methacryloxypropyl-polydimethylsiloxane mit T-förmiger Struktur), (Meth)acrylpolyetheralkylpolydimethylsiloxane (z.B. α-Butyldimethylsiloxy-ω-(3-methacryloxypolyethylenoxidpropylypolydimethylsiloxan), oder Polydimethylsiloxanmonoalkene, besonders bevorzugt Polydimethylsiloxanmono(meth)acrylate, eingesetzt. Hierdurch werden Copolymere erhalten, die als Additive gut zur Verringerung der Oberflächenspannung in Beschichtungsmitteln und Kunststoffen bzw. zur Erzielung kratzfester Oberflächen mit langanhaltendem Anti-Fingerprint-Effekt und schmutzabweisenden Eigenschaften eingesetzt werden können.

Bevorzugt werden fluorierte Comonomere eingesetzt. Hierdurch werden Copolymere erhalten, die als Additive ganz besonders gut als Verlaufmittel, zur Verringerung der Oberflächenspannung in Beschichtungsmitteln und Kunststoffen eingesetzt werden können bzw. zur Erzielung schmutzabweisender Oberflächen eingesetzt werden können.

Bevorzugt werden Ester der (Meth)Acrylsäure mit Alkanolen, beispielsweise mit C1-bis C14-Monohydroxyalkanen, als Comonomere eingesetzt. Hierdurch werden Copolymere erhalten, die als Additive ganz besonders gut zur Verbesserung des Verlaufs, des Glanzes und/oder der Opaleszenz der Beschichtungsmittel bzw. Beschichtungen oder Kunststoffe eingesetzt werden können. Es können beispielsweise n-Butylacrylat, i-Butylacrylat, t-Butylacrylat, Methylacrylat, Ethylacrylat, Propylacrylat, Hexylacrylat, 2-Ethylhexylacrylat, Cyclohexylacrylat, Laurylacrylat, Tetradecylacrylat, Isobornylacrylat, n-Butylmethacrylat, i-Butylmethacrylat, t-Butylmethacrylat, Methylmethacrylat, Ethylmethacrylat, Propylmethacrylat, Hexylmethacrylat, 2-Ethylhexylmethacrylat, Cyclohexylmethacrylat, Laurylmethacrylat, Tetradecylmethacrylat und/oder Isobornylmethacrylat eingesetzt werden. Weiterhin bevorzugte Monomere sind Hydroxyethylacrylat, Hydroxybutylacrylat, Hydroxyethylmethacrylat, Hydroxybutylmethacrylat, 2-Methoxyethylacrylat, 2-Methoxyethylmethacrylat, Styrol, Ethylvinylether, Butylvinylether, Hydroxybutylvinylether, Methacrylsäure und Acrylsäure.

Für die Herstellung von Netz- und Dispergiermitteln, Haftvermittlern und Emulgatoren sind weiterhin folgende Comonomere bevorzugt: Maleinsäureanhydrid, Polyethylenglykolmonomethacrylat, Polypropylenglykolmonomethacrylat, N,N'-Dimethylaminoethylmethacrylat, N,N'-Dimethylaminoethylacrylat, 2-Vinylpyridin, 4-Vinylpyridin, Vinylimidazol.

Bevorzugt können auch Mischungen der oben genannten bevorzugten Comonomere sowie Mischungen dieser Comonomere mit anderen Comonomeren eingesetzt werden.

Die Schreibweise (Meth)acrylat umfasst sowohl Acrylate als auch Methacrylate.

Ionische Gruppen können als entsprechendes ionisches, ethylenisch ungesättigtes Monomer in das Polymer eingeführt werden oder nachträglich durch polymeranaloge Reaktionen, wie zum Beispiel Salzbildung oder Quaternisierung von tertiären Aminoverbindungen, erzeugt werden.

So ist es zum Beispiel möglich, Säurefunktionen im Polymer, wie zum Beispiel Carbonsäuren und Phosphorsäureester, mit Basen umzusetzen. Weiterhin ist es möglich, ausgehend von Säureanhydriden, wie zum Beispiel Maleinsäureanhydrid, zuerst durch Hydrolyse mit Wasser oder Bildung eines Mono- oder Partialesters mit einwertigen Alkoholen oder Polyethern die Carbonsäurefunktion zu generieren und diese anschließend mit Basen umzusetzen.

Oxiranstrukturen im Polymer lassen sich mit Nucleophilen wie o-Phosphorsäure und anschließender Versalzung mit Basen zu ionischen Gruppen umsetzen.

Hydroxyfunktionalitäten im Polymer lassen sich mit Polyphosphorsäure zu Phosphorsäureester und anschließender Versalzung mit Basen zu ionischen Gruppen umsetzen.

Geeignete Basen sind beispielsweise Amine wie zum Beispiel Dimethylaminoethanol, Diethanolamin, Triethanolamin, 2-(Dimethylamino)propan-l-ol, Triethylamin, Butylamin und Dibutylamin, Hydroxide, Oxide, Carbonate und Hydrogencarbonate von Metallen der 1. bis 3. Hauptgruppe des Periodensystems der Elemente, wie zum Beispiel Natriumhydroxid, Kaliumhydroxid, Aluminiumhydroxid und Natriumhydrogencarbonat.

Wie beispielsweise in US 6111054 beschrieben, ist es auch möglich, die Versalzung von am Polymer gebundenen Aminen mit Carbonsäuren oder Phosphorsäuren und deren Estern vorzunehmen.

Weiterhin lassen sich Amine in Alkylierungsreaktionen mit Alkylhalogeniden, wie zum Beispiel mit Benzylchlorid, oder mit einer Kombination von Oxiran mit Carbonsäure in quartäre Ammoniumsalze überführen.

Tertiäre Amine können mit Sauerstoff, Peroxoverbindungen wie Percarbonsäuren und mit Wasserstoffperoxid in Aminoxide überführt werden, die zusätzlich mit Säuren wie zum Beispiel Salzsäure versalzt werden können.

Beispiele für ionische, ethylenisch ungesättigte Monomere können der nachfolgenden Liste entnommen werden, wobei die Schreibweise (Meth)acrylat sowohl Acrylate als auch Methacrylate einschließt: Salze der Acrylsäure, Methacrylsäure oder Maleinsäure; quartäre Aminoalkyl(meth)acrylate, wie zum Beispiel 2-Trimethylammoniumethylmethacrylatchlorid und 2-Benzyldimethyl-ammoniumethyl-(meth)acrylatchlorid; Salze phosphorsäurehaltiger Monomere, wie zum Beispiel Natriumtripropylenglycolmethacrylatphosphat.

Durch beispielsweise die Verwendung von Epoxy-funktionellen Comonomeren, z. B. Glycidylacrylat und Glycidylmethacrylat, oder durch Silan-funktionelle Comonomere können kontrolliert selbstvernetzende Polymere dargestellt werden.

Die Polymere können Homo- oder Copolymere sein. Vorzugsweise sind die Polymere Copolymere.

Werden zur radikalischen Polymerisierung des oder der erfindungsgemäßen oxetan-basierten Makromonomere auch ein oder mehrere Comonomere eingesetzt, so beträgt der Anteil der Einheiten des oder der erfindungsgemäßen oxetan-basierten Makromonomere im entstehenden Copolymer vorzugsweise 1 bis 60 Mol%, bevorzugt von 2 bis 30 Mol%, ganz besonders bevorzugt von 2 bis 15 Mol% Makromonomer-Einheiten, jeweils bezogen auf das gesamte Copolymer.

Die Herstellung der Polymere durch radikalische Polymerisation kann in dem Fachmann bekannter Weise in organischen Lösemitteln oder in Masse in Anwesenheit von Radikalstartern, wie z.B. Peroxiden oder Azoverbindungen, erfolgen. Als Lösemittel kommen insbesondere Ester wie z. B. Ethylacetat, n-Butylacetat oder 1-Methoxy-2-propylacetat sowie aromatische Lösemittel, wie z. B. Toluol oder Xylol und Ketone, wie z. B. Methylisobutylketon oder Methylethylketon, in Frage. Die Wahl des Lösemittels richtet sich auch nach dem späteren Einsatzzweck des Polymeren. Bevorzugt werden niedrigsiedende Lösemittel eingesetzt, um das Abdestillieren dieser Lösemittel zu erleichtern bei Anwendungen, in denen die Polymere als 100%-iges Produkt eingesetzt werden sollen, wie z. B. in UV-härtenden Lacksystemen oder in Polymeren.

Die radikalische Polymerisation wird bei Temperaturen von ca. 40°C bis 180°C, bevorzugt bei 60°C bis 150°C, besonders bevorzugt bei 80°C bis 130°C durchgeführt.

Die radikalische Polymerisation kann als kontinuierliches oder diskontinuierliches Verfahren durchgeführt werden.

Die radikalische Polymerisation kann beispielsweise als Massepolymerisation, als Lösungspolymerisation, als Fällungspolymerisation, als Emulsionspolymerisation oder als Suspensionspolymerisation durchgeführt werden.

Die radikalische Polymerisation kann als freie radikalische Polymerisation oder auch als kontrollierte radikalische Polymerisation durchgeführt werden.

Durch Methoden der kontrollierten radikalischen Polymerisation lassen sich besser definierte Polymerarchitekturen mit einer engeren Molekulargewichtsverteilung erzielen. Es können die dem Fachmann bekannten Methoden der kontrollierten radikalischen Polymerisation verwendet werden, wie beispielsweise ATPR (Atom Transfer Radical Polymerization), GTP (Group Transfer Polymerization), NMP (Nitroxide Mediated Polymerization), RAFT (Reversible Addition Fragmentation Chain Transfer Process) oder MADIX (Macromolecular Design via the Interchange of Xanthates).

Zu den kontrollierten Polymerisationsverfahren gehört insbesondere der "Reversible Addition Fragmentation Chain Transfer Process" (RAFT), der bei Verwendung bestimmter Polymerisationsregler auch "MADIX" (Macromolecular Design via the Interchange of Xanthates) und "Addition Fragmentation Chain Transfer" genannt wird. RAFT ist beispielsweise in Polym. Int. 2000, 49, 993, Aust. J. Chem 2005, 58, 379, J. Polym. Sci. Part A: Polym. Chem. 2005, 43, 5347, Chem. Lett. 1993, 22, 1089, J. Polym. Sci., Part A 1989, 27, 1741 sowie 1991, 29, 1053 sowie 1993, 31, 1551 sowie 1994, 32, 2745 sowie 1996, 34, 95 sowie 2003, 41, 645 sowie 2004, 42, 597 sowie 2004, 42, 6021 und auch in Macromol. Rapid Commun. 2003, 24, 197 und in US 6 291 620, WO 98/01478, WO 98/58974 und WO 99/31144 beschrieben.

Ein weiteres Verfahren zur kontrollierten Polymerisation bedient sich Nitroxylverbindungen als Polymerisationsreglern (NMP) und wird beispielsweise in Chem. Rev. 2001, 101, 3661 offenbart.

Ein weiteres kontrolliertes Polymerisationsverfahren ist auch die "Group Transfer Polymerization" (GTP), wie sie beispielsweise von O. W. Webster in "Group Transfer Polymerization", in "Encyclopedia of Polymer Science and Engineering", Band 7, H. F. Mark, N. M. Bikales, C. G. Overberger and G. Menges, Eds., Wiley Interscience, New York 1987, Seite 580 ff., sowie in O.W. Webster, Adv. Polym. Sci. 2004, 167, 1-34 offenbart ist.

Die kontrollierte radikalische Polymerisation mit Tetraphenylethan, wie sie beispielsweise in Macromol. Symp. 1996, 111, 63 beschrieben ist, ist ein weiteres Beispiel für kontrollierte Polymerisationen.

Die kontrollierte radikalische Polymerisation mit 1,1-Diphenylethen als Polymerisationsregler ist beispielsweise in Macromolecular Rapid Communications, 2001, 22, 700 beschrieben.

Die kontrollierte radikalische Polymerisation mit Inifertern ist beispielsweise in Makromol. Chem. Rapid. Commun. 1982, 3, 127 offenbart.

Die kontrollierte radikalische Polymerisation mit Organokobaltkomplexen, ist beispielsweise aus J. Am. Chem. Soc. 1994, 116, 7973, aus Journal of Polymer Science: Part A: Polymer Chemistry, Vol. 38, 1753-1766 (2000), aus Chem. Rev. 2001, 101, 36113659 sowie aus Macromolecules 2006, 39, 8219-8222 bekannt.

Als weitere kontrollierte Polymerisationstechnik ist der Degenerative Chain Transfer mit Iod-Verbindungen wie zum Beispiel in Macromolecules 2008, 41, 6261 oder in US 7 034 085 beschrieben, anzusehen.

Die kontrollierte radikalische Polymerisation in Gegenwart von Thioketonen wird beispielsweise in Chem. Commun., 2006, 835-837 und in Macromol. Rapid Commun. 2007, 28, 746-753 beschrieben.

Das Polymer kann ein Homopolymer oder ein Copolymer sein. Die Copolymere können statistische Copolymere, Block-Copolymere oder Gradientenpolymere sein und aus zwei oder mehreren, hydrophilen und/oder hydrophoben Monomeren aufgebaut sein.

Das zahlenmittlere Molekulargewicht der Polymere liegt im Bereich von 1500 bis 200000, bevorzugt 5000 bis 75000, besonders bevorzugt im Bereich von 7500 bis 50000. Das zahlenmittlere Molekulargewicht der Polymere wird mittels Gelpermeationschromatographie mit Tetrahydrofuran als Elutionsmittel und unter Verwendung von Polystyrol-Standards bestimmt.
Um das zahlenmittlere Molekulargewicht und die Molekulargewichtsverteilung der Polymere zu beeinflussen, können geeignete Kontroll- bzw. Kettentransfer-Reagenzien eingesetzt werden. Beispielsweise sind hierzu auch Thiole, wie n-Octylmercaptan, n-Dodecylmercaptan oder t-Dodecylmercaptan, und Dimere des alpha-Methylstyrols geeignet. Beispielsweise können bei der Polymerisation geringe Mengen von difunktionellen Monomeren (z.B. Hexandioldiacrylat) mit eingesetzt werden, um das Molgewicht gezielt zu erhöhen.
Die Polymere können durch polymeranaloge Reaktionen noch nachträglich modifiziert werden. Beispielsweise kann durch die nachträgliche Reaktion mit Maleinsäureanhydrid eine reaktive Doppelbindung und Säurefunktion mit eingebaut werden. Diese Säurefunktion kann zur besseren Wasserlöslichkeit auch z.B. mit Triethanolamin versalzen werden. Weiterhin können durch die nachträgliche Umesterung (chemische oder enzymatische Umesterung) mit hydroxyfunktionellen (Meth)acrylaten Produkte erhalten werden, die sich auch bei strahlenhärtenden Prozessen wie UV- und Elektronenstrahlhärtung fest in Lacksysteme einbauen lassen. Auch können freie OH-Gruppen durch z. B. nachträgliche Umsetzung mit Essigsäureanhydrid verestert werden, um bei Anwendung der Polymere als Verlaufshilfsmittel in Lacken potentielle Zwischenschichthaftungsprobleme vermeiden zu können.

### Verwendung der Polymere als Additive

Die Polymere können beispielsweise als Additive in Beschichtungsmitteln oder Kunststoffen verwendet werden. Unter Kunststoffen werden polymere Formmassen und Thermoplasten verstanden.

Die Polymere können zum Beispiel als Verlaufmittel in Beschichtungsmitteln eingesetzt werden, um beispielsweise die optischen Eigenschaften der resultierenden Beschichtung zu verbessern. Durch die Verwendung dieser Polymere als Verlaufmittel kann beispielsweise der Verlauf, der Glanz und/oder die Opaleszenz der Beschichtungsmittel bzw. Beschichtungen oder Kunststoffe verbessert werden.

Die Polymere können zum Beispiel auch eingesetzt werden, um die Oberflächeneigenschaften von Beschichtungen, polymeren Formmassen und Thermoplasten zu verändern. Durch den Zusatz der Polymere kann die Oberflächenenergie von Beschichtungen, polymeren Formmassen und Thermoplasten beeinflusst werden. Die Oberfläche kann hydrophiler oder auch hydrophober gestaltet werden, so dass die Haftung auf dieser Oberfläche verbessert wird oder aber auch vermindert wird, wodurch schmutzabweisende, leicht zu reinigende Oberflächen erhalten werden können. Im allgemeinen werden durch eine Erhöhung der Oberflächenenergie hydrophilere Oberflächen erzielt, die leichter benetzt werden können und bessere Haftungsbedingungen bieten. Dagegen werden im allgemeinen durch eine Verminderung der Oberflächenenergie hydrophobere Oberflächen erzielt, die schwieriger benetzt werden können und schmutzabweisende, anti-adhäsive Eigenschaften aufweisen. Polymere, die zur Erhöhung der Oberflächenenergie geeignet sind, können beispielsweise durch Polymerisation von vergleichsweise hydrophilen erfindungsgemäßen Makromonomeren (z.B. auf Basis von ethoxyliertem TMPO) oder durch Copolymerisation mit eher hydrophilen Comonomeren (z.B. hydrophile Acrylate wie Polyethylenoxid-(Meth)acrylate oder (Meth)acrylsäure) erhalten werden. Polymere, die zur Verminderung der Oberflächenenergie geeignet sind, können beispielsweise durch Polymerisation von vergleichsweise hydrophoben erfindungsgemäßen Makromonomeren (z.B. auf Basis von TMPO oder propoxyliertem TMPO) oder durch Copolymerisation mit eher hydrophoben oder besonders geeigneten Comonomeren, wie z.B. Perfluorogruppen und/oder Polysiloxangruppen enthaltenden Comonomeren, erhalten werden.

### Additive zur Erzielung schmutzabweisender, anti-adhäsiver Oberflächen

Die Copolymere, die Beschichtungsmitteln, polymeren Formmassen und Thermoplasten zugesetzt werden können und durch deren Zusatz schmutzabweisende, leicht zu reinigende, anti-adhäsive Oberflächen erzielt werden können, sind beispielsweise herstellbar über die Polymerisation mindestens eines erfindungsgemäßen Makromonomers oder die Copolymerisation mindestens eines erfindungsgemäßen Makromonomers und mindestens eines Comonomers, beispielsweise eines Perfluorogruppen und/oder Polysiloxangruppen enthaltenden Comonomers.

Beschichtungsmittel, polymere Formmassen und Thermoplasten, denen entsprechende Copolymere zugesetzt werden, weisen ausgezeichnete anti-adhäsive und schmutzabweisende Eigenschaften auf. Die Copolymere beeinträchtigen auch die anderen Eigenschaften der Beschichtungsmittel bzw. Beschichtungen, polymeren Formmassen oder Thermoplasten nicht wesentlich. Dabei können diese Copolymere den Beschichtungsmitteln, polymeren Formmassen oder Thermoplasten in relativ geringen Mengen (Additivmengen) zugesetzt werden. Insbesondere in Beschichtungsmitteln und Kunststoffen, wie polymeren Formmassen und Thermoplasten, können die Polymere vorzugsweise auch in relativ geringen Mengen von 0,01 bis 5 Gew.-%, bevorzugt von 0,05 bis 2 Gew.-% und besonders bevorzugt von 0,1 bis 1 Gew.-%, jeweils bezogen auf das gesamte Beschichtungsmittel oder den gesamten Kunststoff, eingesetzt werden.

Die physikalischen Eigenschaften der ursprünglichen Beschichtungsmittel bzw. Beschichtungen, polymeren Formmassen und Thermoplasten beispielsweise in Bezug auf Korrosionsschutz, Glanzhaltung und Witterungsbeständigkeit werden durch die geringen Konzentrationen des Additivs nicht beeinträchtigt. Beschichtungsmittel bzw. Beschichtungen, polymere Formmassen und Thermoplasten, die die Copolymere enthalten, zeigen die gewünschten Eigenschaften in der Regel auch über einen Zeitraum von mehreren Jahren hinweg und behalten diese Eigenschaften auch über mehrere Reinigungszyklen hinweg bei.

Weiterhin zeigte es sich als besonders vorteilhaft, dass die Hydroxylgruppen der oxetan-basierten Makromonomer-Einheiten in den Polymeren mit reaktiven Gruppen des Bindemittels vernetzen können und somit einen dauerhaften Effekt gewährleisten.

Durch die Verwendung der Polymere als Additive in Beschichtungsmitteln, polymeren Formmassen oder Thermoplasten können auch Oberflächen mit antistatischen Eigenschaften oder Antibeschlag-Eigenschaften erhalten werden.

### Additive zur Erzielung leicht benetzbarer Oberflächen

Die Copolymere können Beschichtungsmitteln, polymeren Formmassen und Thermoplasten zugesetzt werden, so dass durch deren Zusatz die Oberflächenenergie der Beschichtungen, polymeren Formmassen und Thermoplasten erhöht wird, so dass die Benetzbarkeit dieser Oberflächen verbessert wird.

Beschichtungen, polymere Formmassen und Thermoplasten, denen entsprechende Copolymere zugesetzt werden, weisen ausgezeichnet benetzbare Oberflächen auf. Die Benetzbarkeit kann bestimmt werden, indem man nach den üblichen Methoden den Kontaktwinkel der Oberfläche gegen Wasser bestimmt. Für hydrophile Oberflächen soll der Kontaktwinkel < 60° betragen. Die Copolymere beeinträchtigen auch die anderen Eigenschaften der Beschichtungsmittel bzw. Beschichtungen, polymeren Formmassen oder Thermoplasten nicht wesentlich. Dabei können diese Copolymere den Beschichtungsmitteln, polymeren Formmassen oder Thermoplasten in relativ geringen Mengen (Additivmengen) zugesetzt werden, beispielsweise in Mengen von 0,01 bis 5 Gew.%, bevorzugt von 0,05 bis 2 Gew.-% und besonders bevorzugt von 0,1 bis 1 Gew.-%, jeweils bezogen auf das gesamte Beschichtungsmittel oder den gesamten Kunststoff. Die physikalischen Eigenschaften der ursprünglichen Beschichtungsmittel bzw. Beschichtungen, polymeren Formmassen und Thermoplasten beispielsweise in Bezug auf Korrosionsschutz, Glanzhaltung und Witterungsbeständigkeit werden durch die geringen Konzentrationen des Additivs nicht beeinträchtigt. Beschichtungsmittel bzw. Beschichtungen, polymere Formmassen und Thermoplasten, die die Copolymere enthalten, zeigen die gewünschten Eigenschaften in der Regel auch über einen Zeitraum von mehreren Jahren hinweg und behalten diese Eigenschaften auch über mehrere Reinigungszyklen hinweg bei.

Weiterhin zeigte es sich als besonders vorteilhaft, dass die Hydroxylgruppen der oxetan-basierten Makromonomer-Einheiten in den Polymeren mit reaktiven Gruppen des Bindemittels vernetzen können und somit einen dauerhaften Effekt gewährleisten.

### Netz- und Dispergiermittel

Die Polymere können auch als Dispergiermittel eingesetzt werden, und zwar in den aus dem Stand der Technik bekannten Einsatzgebieten von Dispergiermitteln, wobei anstelle der oder zusammen mit den aus dem Stand der Technik bekannten Dispergiermitteln die Polymere als Dispergiermittel eingesetzt werden. So können diese z. B. bei der Herstellung oder Verarbeitung von Lacken, Druckfarben, Papierstrich, Leder- und Textilfarben, Pasten, Pigmentkonzentraten, Keramiken oder kosmetischen Zubereitungen eingesetzt werden, insbesondere wenn diese Feststoffe wie Pigmente und/oder Füllstoffe enthalten. Auch bei der Herstellung oder Verarbeitung von Gieß- und/oder Formmassen auf Basis von synthetischen, halbsynthetischen oder natürlichen makromolekularen Stoffen, wie zum Beispiel Polyvinylchlorid, gesättigten oder ungesättigten Polyestern, Polyurethanen, Polystyrol, Polyacrylat, Polyamid, Epoxidharzen, Polyolefinen wie Polyethylen oder Polypropylen können sie eingesetzt werden. Beispielsweise lassen sich die Copolymere zur Herstellung von Gießmassen, PVC-Plastisolen, Gelcoats, Polymerbeton, Leiterplatten, Industrielacken, Holz- und Möbellacken, Fahrzeuglacken, Schiffsfarben, Korrosionsschutzfarben, Can- und Coil-Coatings, Maler- und Bautenlacken, wobei Bindemittel und/oder Lösemittel, Pigmente und gegebenenfalls Füllstoffe, die Kammcopolymere und übliche Hilfsstoffe vermischt werden, verwenden. Beispiele für übliche Bindemittel sind Harze auf Basis von Polyurethan, Cellulosenitrat, Celluloseacetobutyrat, Alkyd, Melamin, Polyester, Chlorkautschuk, Epoxid und Acrylat. Beispiele für Beschichtungen auf Wasserbasis sind kathodische oder anodische Elektrotauchlackierungen zum Beispiel für Automobilkarossen. Weitere Beispiele sind Putze, Silikatfarben, Dispersionsfarben, Wasserlacke auf Basis von wasserverdünnbaren Alkyden, Alkydemulsionen, Hybridsystemen, 2-Komponenten-Systemen, Polyurethan- und Acrylat-Dispersionen.

Die Copolymere eignen sich insbesondere auch als Dispergiermittel zur Herstellung von Feststoffkonzentraten, wie zum Beispiel Pigmentkonzentraten. Dazu werden beispielsweise die Copolymere in einem Trägermedium wie organischen Lösemitteln, Weichmachern und/oder Wasser vorgelegt, und die zu dispergierenden Feststoffe werden unter Rühren zugegeben. Zusätzlich können diese Konzentrate Bindemittel und/oder andere Hilfsstoffe enthalten. Mit den Copolymeren ist es aber insbesondere möglich, stabile bindemittelfreie Pigmentkonzentrate herzustellen. Ebenso ist es möglich, mit den Polymeren fließfähige Feststoffkonzentrate aus Pigmentpresskuchen herzustellen. Hierbei werden dem Presskuchen, der noch organische Lösemittel, Weichmacher und/oder Wasser enthalten kann, die Copolymere zugemischt und die so erhaltene Mischung dispergiert. Die auf verschiedenen Wegen hergestellten Feststoffkonzentrate können dann in unterschiedliche Substrate wie z. B. Alkydharze, Polyesterharze, Acrylatharze, Polyurethanharze oder Epoxidharze eingearbeitet werden. Pigmente können aber auch lösemittelfrei direkt in den Copolymeren dispergiert werden und eignen sich dann besonders zur Pigmentierung von thermoplastischen und duroplastischen Kunststoffformulierungen.

Die Copolymere können vorteilhaft auch bei der Herstellung von Tinten für "non impact"-Druckverfahren wie "thermal inkjet" und dem "Bubblejet-Verfahren" verwendet werden. Diese Tinten können beispielsweise wässrige Tintenformulierungen, lösemittelbasierte Tintenformulierungen, lösemittelfreie oder - arme Tinten für UV-Applikationen als auch wachsartige Tinten sein.

Die Copolymere können vorteilhaft auch bei der Herstellung von Farbfiltern für Flüssigkristallanzeigen, -bildschirme, Farbauflösungsgeräte, Sensoren, Plasmabildschirme, Anzeigen auf Basis der SED (Surface conduction Electron emitter Display) und für MLCC (Multi Layer Ceramic Compounds) verwendet werden. Hierbei kann der flüssige Farbfilterlack, der auch Color Resist genannt wird, durch verschiedenste Applikationsverfahren wie Spin Coating, Aufrakeln, Kombination beider oder über "non impact"-Druckverfahren wie zum Beispiel Inkjet-Verfahren aufgetragen werden. Die MLCC-Technologie wird bei der Herstellung von Mikrochips und Leiterplatten verwendet.

Die Copolymere können auch zur Herstellung kosmetischer Zubereitungen wie zum Beispiel Make-up, Puder, Lippenstifte, Haarfärbemittel, Cremes, Nagellacke und Sonnenschutzpräparate verwendet werden. Diese können in den üblichen Formen, wie beispielsweise als W/O- oder O/W-Emulsionen, Lösungen, Gele, Cremes, Lotionen oder Sprays, vorliegen. Die Copolymere können vorteilhaft in zur Herstellung dieser Zubereitungen verwendeten Dispersionen eingesetzt werden. Diese können die für diese Zwecke in der Kosmetik üblichen Trägermedien, wie zum Beispiel Wasser, Ricinusöle oder Silikonöle und Feststoffe, wie zum Beispiel organische und anorganische Pigmente wie Titandioxid oder Eisenoxid, enthalten.

Ein derartiges Dispergiermittel kann schließlich auch zur Herstellung eines pigmentierten Überzuges auf einem Substrat verwendet werden, wobei der Pigmentlack auf das Substrat aufgebracht wird und wobei der auf das Substrat aufgebrachte Pigmentlack eingebrannt oder ausgehärtet bzw. vernetzt wird.

Die Copolymere können alleine oder zusammen mit nach dem Stand der Technik üblichen Bindemitteln eingesetzt werden. Beim Einsatz in Polyolefinen kann es z. B. vorteilhaft sein, entsprechende niedermolekulare Polyolefine als Trägermaterialien zusammen mit dem Kammcopolymer einzusetzen.

Eine mögliche Verwendung der Copolymere besteht auch in der Herstellung dispergierbarer pulverpartikel- und/oder faserpartikelförmiger Feststoffe, insbesondere von dispergierbaren Pigmenten oder Kunststofffüllstoffen, wobei die Partikel mit dem Kammcopolymer beschichtet sind. Derartige Beschichtungen von organischen wie auch anorganischen Feststoffen werden in bekannter Weise ausgeführt, wie sie z. B. in EP-A-0 270 126 beschrieben werden. Hierbei kann das Lösungs- oder Emulsionsmittel entweder entfernt werden oder im Gemisch unter Bildung von Pasten verbleiben. Diese Pasten sind übliche Handelsprodukte und können zusätzlich Bindemittelanteile sowie weitere Hilfs- und Zusatzstoffe enthalten. Speziell bei Pigmenten kann die Beschichtung der Pigmentoberfläche während oder nach der Synthese der Pigmente erfolgen, z. B. durch Zusatz der Copolymere zur Pigmentsuspension oder während oder nach dem Pigmentfinish. Die auf diese Weise vorbehandelten Pigmente zeichnen sich durch leichtere Einarbeitbarkeit sowie durch verbessertes Viskositäts-, Flockulations- und Glanzverhalten und durch höhere Farbstärke gegenüber nicht behandelten Pigmenten aus.

Beispiele für Pigmente sind Mono-, Di-, Tri- und Polyazopigmente, Oxazin-, Dioxazin-, Thiazin-Pigmente, Diketo-pyrrolo-pyrrole, Phthalocyanine, Ultramarin und andere Metallkomplex-Pigmente, indigoide Pigmente, Diphenylmethan-, Triarylmethan-, Xanthen-, Acridin-, Chinacridon-, Methin-Pigmente, Anthrachinon, Pyranthron-, Perylen- und andere polycyclische Carbonyl-Pigmente. Weitere Beispiele für organische Pigmente finden sich in der Monographie: W. Herbst, K. Hunger "Industrial Organic Pigments", 1997 (Verlag: Wiley-VCH, ISBN: 3-527-28836-8). Beispiele für anorganische Pigmente sind Pigmente auf Basis von Ruß, Graphit, Zink, Titandioxid, Zinkoxid, Zinksulfid, Zinkphosphat, Bariumsulfat, Lithophone, Eisenoxid, Ultramarin, Manganphosphat, Cobaltaluminat, Cobaltstannat, Cobaltzinkat, Antimonoxid, Antimonsulfid, Chromoxid, Zinkchromat, gemischten Metalloxiden auf Basis von Nickel, Bismut, Vanadium, Molybdän, Cadmium, Titan, Zink, Mangan, Cobalt, Eisen, Chrom, Antimon, Magnesium, Aluminium (beispielsweise Nickeltitangelb, Bismut-vanadat-molybdatgelb oder Chromtitangelb), Weitere Beispiele sind in der Monographie: G. Buxbaum "Industrial Inorganic Pigments", 1998 (Verlag: Wiley-VCH, ISBN: 3-527-28878-3) genannt. Anorganische Pigmente können auch magnetische Pigmente auf Basis von Reineisen, Eisenoxiden und Chromoxiden oder Mischoxyden, Metalleffektpigmente aus Aluminium, Zink, Kupfer oder Messing sowie Pedglanzpigmente, fluoreszierende und phosphoreszierende Leuchtpigmente sein. Weitere Beispiele sind nanoskalige organische oder anorganische Feststoffe mit Teilchengrößen unterhalb von 100 nm, wie bestimmte Rußtypen oder Partikel, die aus einem Metall-, oder Halbmetalloxid bzw. -hydroxid bestehen, sowie Partikel, die aus gemischten Metall- und/oder Halbmetalloxiden bzw. -hydroxiden bestehen. Beispielsweise lassen sich die Oxide und/oder Oxidhydroxide des Aluminiums, Siliziums, Zinks, Titans, usw. zur Herstellung solcher extrem feinteiligen Feststoffe heranziehen. Der Herstellprozess dieser oxidischen bzw. hydroxidischen bzw. oxidhydroxidischen Teilchen kann über die unterschiedlichsten Verfahren wie beispielsweise Ionenaustauschprozesse, Plasma prozesse, Sol-Gel-Verfahren, Ausfällung, Zerkleinerung (z. B. durch Vermahlung) oder Flammhydrolyse usw. erfolgen. Bei diesen nanoskaligen Feststoffen kann es sich auch um sog. Hybrid-Partikel handeln, die aus einem anorganischen Kern und einer organischen Hülle - oder umgekehrt - bestehen.

Beispiele für pulver- oder faserförmige Füllstoffe sind zum Beispiel solche, die aufgebaut sind aus pulver- oder faserförmigen Teilchen von Aluminiumoxid, Aluminiumhydroxid, Siliziumdioxid, Kieselgur, Kieselerde, Quarz, Kieselgel, Talkum, Kaolin, Glimmer, Perlite, Feldspat, Schiefermehl, Calciumsulfat, Bariumsulfat, Calciumcarbonat, Calcit, Dolomit, Glas oder Kohlenstoff. Weitere Beispiele für Pigmente oder Füllstoffe finden sich beispielsweise in der EP-A-0 270 126. Auch Flammschutzmittel wie z. B. Aluminium- oder Magnesiumhydroxid und Mattierungsmittel wie z. B. Kieselsäuren lassen sich ebenfalls hervorragend dispergieren und stabilisieren.

Die Copolymere können auch als Emulgator für Emulsionen eingesetzt werden. Da Emulsionen üblicherweise instabile Systeme sind, die sich nicht spontan bilden, sondern erst durch Schütteln, Rühren, Homogenisierung oder Sprühprozessen eine Verteilung der Phasen ineinander erhalten wird, werden Emulgatoren eingesetzt, um diese instabilen Strukturen zu stabilisieren. Der Einsatz von Emulgatoren vermeidet eine Separierung der Phasen.

Die Copolymere können auch als Rheologie-Additive eingesetzt werden.

Die Copolymere können auch als Haftvermittler eingesetzt werden.

Die Polymere können in den Beschichtungsmitteln, polymeren Formmassen, Thermoplasten oder sonstigen Zusammensetzungen in relativ breiten Mengen von 0,01 bis 100 Gew.-%, bevorzugt 0,05 bis Gew.-50 %, ganz besonders bevorzugt 0,1 bis 40 Gew.-%, jeweils bezogen auf das gesamte Beschichtungsmittel oder den gesamten Kunststoff, eingesetzt werden.

Insbesondere in Beschichtungsmitteln und Kunststoffen, wie polymeren Formmassen und Thermoplasten, können die Polymere vorzugsweise auch in relativ geringen Mengen von 0,01 bis 5 Gew.-%, bevorzugt von 0,05 bis 2 Gew.-% und besonders bevorzugt von 0,1 bis 1 Gew.-%, jeweils bezogen auf das gesamte Beschichtungsmittel oder den gesamten Kunststoff, eingesetzt werden.

Die Polymere können als Lösungen, Emulsionen oder als 100%-ige Stoffe eingesetzt werden, abhängig von der Art und Applikationsweise des Beschichtungsmittels, der polymeren Formmasse, des Thermoplasten oder der sonstigen Zusammensetzung.

### BEISPIELE

### Abkürzungen:

"TMPO" = 3-Ethyl-3-(hydroxymethyl)oxetan
   "TMPO-EOX" = 3-Ethyl-3-(hydroxymethyl)oxetan, modifiziert mit im Durchschnitt 3,3 Mol Ethylenoxid
   "HEMA" = 2-Hydroxyethylmethacrylat
   "BA" = Butylacetat
   "EHA" = 2-Ethylhexylacrylat
"IBMA" = Isobutylmethacrylat
   "STY" = Styrol
   "DMAEMA" = 2-(N,N-Dimethylamino)ethylmethacrylat
   "AA" = Acrylsäure
"PMA" = 1-Methoxy-2-propylacetat (z.B. Dowanol PMA)
   Trigonox C (= tert. Butylperbenzoat) ist ein Starter der radikalischen Polymerisation (erhältlich von Akzo Nobel Chemicals).
   Perkadox AMBN (= 2,2'-Azobis(2-methylbutyronitril) ist ein Starter der radikalischen Polymerisation (erhältlich von Akzo Nobel Chemicals).
   Silaplane FM0721 ist ein Polysiloxanmonomethacrylat (Mw-5000) erhältlich von Chisso Corp.
   BLEMMER PME-1000 ist ein lineares Methoxypolyethylenglycol-Monomethacrylat (Mw-1000) erhältlich von NOF CORPORATION.
   BISOMER MPEG550MA ist ein lineares Methoxypolyethylenglycol-Monomethacrylat (Mw - 628) erhältlich von Cognis.

Fluowet MA600 ist ein fluoriertes Methacrylat von Clariant (3,3,4,4,5,5,6,6,7,7,8,8,8,-tridecafluorooctylmethacrylat).
Amberlyst IR120H ist ein Kationenaustauscherharz von Rohm & Haas.
Amberlyst A- 21 ist ein Anionenaustauscherharz von Rohm & Haas.

### Messverfahren:

### Größenausschlußchromatografie (SEC, size exclusion chromatography):

Die Größenausschlußchromatografie wurde bei 40°C mit einer Hochdruckflüssigkeitschromatografie-Pumpe (Bischoff HPLC 2200) und einem Brechungsindexdetektor (Waters 419) durchgeführt. Als Elutionsmittel wurde Tetrahydrofuran (THF) mit einer Elutionsgeschwindigkeit von 1 mL/min verwendet. Die konventionelle Kalibrierung wurde mittels Polystyrol-Standards durchgeführt. Das zahlenmittlere Molekulargewicht Mn, das gewichtsmittlere Molekulargewicht Mw und die Polydispersität Q = Mw/Mn wurden mit dem Programm NTeqGPC berechnet.

### NMR-Spektroskopie:

NMR-Messungen wurden mit einem NMR-Gerät (Bruker DPX 300) bei 300 MHz (¹H) bzw. 75 MHz (¹³C) durchgeführt. Als Lösemittel wurden deuteriertes Chloroform (CDCl₃) und deuteriertes Dimethylsulfoxid (DMSO-*d*₆) verwendet.

### Beispiel 1:

### Synthese der erfindungsgemäßen Makromonomere

### MAKROMONOMER 1

### Herstellung:

100 g HEMA wurden bei Raumtemperatur in ein Reaktionsgefäß gegeben, welches mit einem mechanischen Rührer, einem Zugabetrichter, einem Thermometer, einem Wärmemantel und einem Kühler ausgestattet war. Die Reaktion wurde in normaler Atmosphäre (Luft) durchgeführt.
178,4 g TMPO und 200 g TMPO-EOX wurden in den Zugabetrichter gegeben und dort gemischt. Die Mischung der Oxetan-Monomere war homogen.
240 mg Hydrochinon und 240 mg 2,6-Di-tert.-butyl-4-methylphenol wurden zu dem Startmolekül (HEMA) ins Reaktionsgefäß gegeben. Die Mischung wurde gerührt, bis eine Lösung erhalten wurde. Hierzu wurden 0,36 g Trifluormethansulfonsäure als Katalysator gegeben und die Temperatur auf 80°C erhöht.
Die Mischung der Oxetan-Monomere wurde anschließend kontinuierlich tropfenweise über 8 Stunden hinzugegeben, wobei die Temperatur so kontrolliert wurde, dass sie nicht über 80°C lag. Nach erfolgter Zugabe der Mischung der Oxetan-Monomere wurde das Reaktionsgemisch für weitere 2 Stunden bei 80°C gerührt.
Danach wurde das Reaktionsgemisch auf Raumtemperatur abgekühlt und das viskose Produkt in 160 g PMA gelöst. Der Katalysator wurde mit Amberlyst A-21 neutralisiert und der Ionenaustauscher abfiltriert.

### Analyse des Produkts:

NMR: kein Oxetan vorhanden
Hydroxylzahl: 269 mg KOH/g
Jodzahl: 28,5 g I₂ g/100g

### MAKROMONOMER 2

### Herstellung:

100 g HEMA wurden bei Raumtemperatur in ein Reaktionsgefäß gegeben, welches mit einem mechanischen Rührer, einem Zugabetrichter, einem Thermometer, einem Wärmemantel und einem Kühler ausgestattet war. Die Reaktion wurde in normaler Atmosphäre (Luft) durchgeführt.
267,7 g TMPO wurden in den Zugabetrichter gegeben.
184 mg Hydrochinon und 184 mg 2,6-Di-tert.-butyl-4-methylphenol wurden zu dem Startmolekül (HEMA) ins Reaktionsgefäß gegeben. Die Mischung wurde gerührt, bis eine Lösung erhalten wurde. Hierzu wurden 0,28 g Trifluormethansulfonsäure als Katalysator gegeben und die Temperatur auf 75°C erhöht.
Das Oxetan-Monomer wurde anschließend kontinuierlich tropfenweise über 6 Stunden hinzugegeben, wobei die Temperatur so kontrolliert wurde, dass sie nicht über 80°C lag. Nach erfolgter Zugabe des Oxetan-Monomers wurde das Reaktionsgemisch für eine weitere Stunde bei 80°C gerührt.

Danach wurde das Reaktionsgemisch auf Raumtemperatur abgekühlt und das viskose Produkt in 368 g PMA gelöst. Der Katalysator wurde mit Amberlyst A-21 neutralisiert und der Ionenaustauscher abfiltriert.

### Analyse des Produkts:

NMR: kein Oxetan vorhanden
Hydroxylzahl: 214 mg KOH/g
Jodzahl: 24,9 g I₂ g/100g

### MAKROMONOMER 3

### Herstellung:

In einem 1000-ml-4-Halskolben mit Rührer, Thermometer, Rückflußkühler und Tropftrichter werden bei Raumtemperatur 67,94 g HEMA, 0,375 g 2,6-Di-tert.-butyl-4-methylphenol und 0,375 g Hydrochinon vorgelegt. Unter einem leichten Luftstrom, der während der gesamten Reaktion beibehalten wird, wird die Reaktionsmischung erwärmt. Bei Erreichen einer Temperatur von ca. 40°C werden 0,77 g Trifluormethansulfonsäure zugegeben. Bei Erreichen einer Temperatur von 80°C wird die Dosierung von 682,06 g TMPO-EOX gestartet. Die Dosierung wird in 5,5 h durchgeführt, wobei die Temperatur bei 80°C gehalten wird. Nach einer Nachreaktionszeit von 1 h 45 min wird der Ansatz abgekühlt. 250 g PMA werden zugegeben. Der Katalysator wird durch Zugabe von 3,5 g Amberlyst A21 unter Rühren neutralisiert. Nach 45 min wird der Ionentauscher durch Filtration entfernt. Man erhält eine hellgelbe viskose Flüssigkeit.

### Analyse des Produkts:

NMR: kein Oxetan vorhanden
Hydroxylzahl: 185 mg KOH/g
Jodzahl: 12,3 g I₂ g/100g

### MAKROMONOMER 4

### Herstellung:

In einem 250-ml-4-Halskolben mit Rührer, Thermometer, Rückflußkühler und Tropftrichter werden bei Raumtemperatur 28,50 g HEMA, 0,1 g 2,6-Di-tert.-butyl-4-methylphenol und 0,1 g Hydrochinon vorgelegt. Unter einem leichten Luftstrom, der während der gesamten Reaktion beibehalten wird, wird die Reaktionsmischung erwärmt. Bei Erreichen einer Temperatur von ca. 40°C werden 0,22 g Trifluormethansulfonsäure zugegeben. Bei Erreichen einer Temperatur von 80°C wird die Dosierung von 171,50 g TMPO-EOX gestartet. Die Dosierung wird in 2 h durchgeführt, wobei die Temperatur bei 80°C gehalten wird. Nach einer Nachreaktionszeit von 3 h 30 min werden 50 g Methylisobutylketon zugegeben und der Ansatz auf 60°C abgekühlt. Der Katalysator wird durch Zugabe von 1 g Amberlyst A21 unter Rühren neutralisiert. Nach 1 h bei 60°C wird der Ionentauscher durch Filtration entfernt. Man erhält eine hellgelbe viskose Flüssigkeit.

### Analyse des Produkts:

NMR: kein Oxetan vorhanden.
Hydroxylzahl: 209 mg KOH/g
Jodzahl: 21,4 g I₂ g/100g

### MAKROMONOMER 5

### Herstellung:

### 1. Derivatisierung von TMPO-EOX mit Glycidol zu TMPO-EOX-GI

Die Reaktion wurde unter Stickstoffatmosphäre (Trocknung mit P₂O₅) durchgeführt. 400 g TMPO-EOX wurden in einem 1000 ml-Vierhalskolben vorgelegt. Über einen Tropftrichter wurden 17,59 g Kalium-tert.-Butanolat hinzugegeben und das Gemisch gerührt, bis eine Lösung erhalten wurde. Die leicht orangefarbene Lösung wurde auf 80°C erwärmt. Unter gelegentlicher Anwendung von Unterdruck wurde entstehendes Tert-Butanol während 1 Stunde abdestilliert. Die Temperatur des Reaktionsgemisches wurde bei 80°C gehalten. Zum Reaktionsgemisch wurden dann langsam unter Rühren während 4 Stunden 340,22 g des Glycidol-Monomers hinzugetropft (exotherme Reaktion), wobei die Temperatur des Reaktionsgemisches so kontrolliert wurde, dass sie stets unter 90°C lag. Nach Beendigung der Zugabe wurde das Reaktionsgemisch für eine weitere Stunde bei 80°C gerührt.
Dann wurde eine Probe entnommen und sofort mittels NMR-Spektroskopie analysiert, wobei sich zeigte, dass keine Glycidol-Monomere mehr enthalten waren und der Oxetan-Ring intakt war. Daraufhin wurde die Reaktion beendet und das Reaktionsgemisch auf Raumtemperatur abgekühlt. Es wurde eine viskose, gelbliche Flüssigkeit erhalten, die mit 200 ml. Methanol verdünnt und mit Amberlyst A21 neutralisiert wurde. Das Ionenaustauscherharz wurde abfiltriert. Die Lösemittel wurden unter Vakuum (bis zu 2 mbar bei 50°C) abdestilliert. Die anschließende NMR-Analyse zeigte, dass das Produkt 4 Mol Hydroxygruppen pro 1 Mol Oxetan enthielt.

### 2. Umsetzung von HEMA mit TMPO-EOX und TMPO-EOX-GI

25 g HEMA wurden bei Raumtemperatur in ein Reaktionsgefäß gegeben, welches mit einem mechanischen Rührer, einem Zugabetrichter, einem Thermometer, einem Wärmemantel und einem Kühler ausgestattet war. Die Reaktion wurde in normaler Atmosphäre (Luft) durchgeführt.
150,73 g TMPO-EOX und 92,98 g TMPO-EOX-GI wurden in den Zugabetrichter gegeben.
134 mg Hydrochinon und 134 mg 2,6-Di-tert.-butyl-4-methylphenol wurden zu dem Startmolekül (HEMA) ins Reaktionsgefäß gegeben. Die Mischung wurde gerührt, bis eine Lösung erhalten wurde. Hierzu wurden 0,20 g Trifluormethansulfonsäure als Katalysator gegeben und die Temperatur auf 75°C erhöht.

Die Mischung der Oxetan-Monomere wurde anschließend kontinuierlich tropfenweise über 6 Stunden hinzugegeben, wobei die Temperatur so kontrolliert wurde, dass sie nicht über 80°C lag. Nach erfolgter Zugabe des Oxetan-Monomers wurde das Reaktionsgemisch für eine weitere Stunde bei 80°C gerührt.

Danach wurde das Reaktionsgemisch auf Raumtemperatur abgekühlt und das viskose Produkt in 269 g Isobutanol gelöst. Der Katalysator wurde mit Amberlyst A-21 neutralisiert und der Ionenaustauscher abfiltriert.

### Analyse des Produkts:

NMR: kein Oxetan vorhanden
Hydroxylzahl: 547 mg KOH/g
Jodzahl: 10,0 g I₂ g/100g

### 111. Weitere Derivatisierung von erfindungsgemäßen Makromonomeren

### MAKROMONOMER 6

### Herstellung:

In einem 250-ml-4-Halskolben mit Rührer, Thermometer, Rückflußkühler und Tropftrichter werden bei Raumtemperatur 100 g Makromonomer 2 und 0,2 g einer 10%igen Lösung von Dibutylzinnlaurat (DBTL) in Xylol vorlegt. Unter einem leichten Stickstoffstrom, der während der gesamten Reaktion beibehalten wird, wird die Reaktionsmischung auf 60°C erwärmt. Bei Erreichen der Temperatur werden in 30 min. 100 g Stearylisocyanat zudosiert, so daß die Temperatur durch die freiwerdende Reaktionswärme nicht über 65°C steigt. Nach beendeter Zugabe wird der Ansatz noch 4 h bei 60°C gehalten. Der Ansatz wird beendet.
Eine NCO-Bestimmung ergibt einen vollständigen Umsatz bezogen auf Stearylisocyanat.
Man erhält eine hellgelbe viskose Flüssigkeit, die in der Kälte teilweise erstarrt.

### MAKROMONOMER 7

### Herstellung:

In einem 250-ml-4-Halskolben mit Rührer, Thermometer, Rückflußkühler und Tropftrichter werden bei Raumtemperatur 100 g Makromonomer 4 und 0,3 g einer 10%igen Lösung von Dibutylzinnlaurat (DBTL) in Xylol vorlegt. Unter einem leichten Stickstoffstrom, der während der gesamten Reaktion beibehalten wird, wird die Reaktionsmischung auf 70°C erwärmt. Bei Erreichen der Temperatur werden in 45 min. 200 g MPEG350-TDI-Addukt (herstellt aus der Umsetzung von MPEG350 mit TDI, NCO-Gehalt 7,97%, hergestellt analog Patent DE10039837), so zudosiert, daß die Temperatur durch die freiwerdende Reaktionswärme nicht über 75°C steigt. Nach beendeter Zugabe wird der Ansatz noch 3 h bei 70°C gehalten. Der Ansatz wird beendet.
Eine NCO-Bestimmung ergibt einen vollständigen Umsatz bezogen auf MPEG350-TDI-Addukt.
Man erhält eine gelbe viskose Flüssigkeit.

### Beispiel 2:

### Synthese der Polymere

### POLYMER 1

### Herstellung:

Lösemittel und Monomere wurden durch Stickstoffeinblasung entlüftet. Die Reaktion wurde unter Stickstoffatmosphäre durchgeführt.
Die Polymerisation wurde in einem 500 ml-Vierhalskolben durchgeführt, der mit einer Kühleinheit, einem Heizmantel, Thermometer, mechanischem Rührer und einer Vorrichtung zur Stickstoffeinblasung ausgestattet war.
Das Lösemittel PMA (142,61 g), die Monomere IBMA (14,56 g), STY (9,80 g), Makromonomer 3 (21,14 g) und Silaplane FM0721 (10,63 g) sowie der Radikalstarter Trigonox C (0,61 g) wurden in das Reaktionsgefäß gegeben, gemischt, mit Stickstoff entlüftet und für 3 Stunden auf 130°C erwärmt.
Anschließend wurden weitere 0,1 g des Radikalstarters Trigonox. C hinzugefügt. Nach weiteren 30 Minuten wurden erneut weitere 0,1 g des Radikalstarters hinzugefügt. Die Reaktion wurde weitere 30 Minuten fortgesetzt und das Reaktionsgemisch dann auf Raumtemperatur abgekühlt. Das Lösemittel wurde unter vermindertem Druck abdestilliert.

### Analyse des Produkts (SEC):

Mw = 14899
Polydispersität 4,47

### POLYMER 2

### Herstellung:

Lösemittel und Monomere wurden durch Stickstoffeinblasung entlüftet. Die Reaktion wurde unter Stickstoffatmosphäre durchgeführt.
Die Polymerisation wurde in einem 500 ml-Vierhalskolben durchgeführt, der mit einer Kühleinheit, einem Heizmantel, Thermometer, mechanischem Rührer und einer Vorrichtung zur Stickstoffeinblasung ausgestattet war.
Das Lösemittel PMA (142,61 g) und das Monomer Silaplane FM0721 (10,50 g) wurden in das Reaktionsgefäß gegeben, gemischt, mit Stickstoff entlüftet und auf 130°C erwärmt.
Die weiteren Monomere (IBMA (14,10 g), STY (9,68 g), Makromonomer 3 (20,89 g), Fluowet MA600 (0,89 g)) sowie der Radikalstarter (Trigonox C (0,60 g)) und das Kettentransfer-Reagenz (alpha-Methylstyrol-Dimer (0,73 g)) wurden gemischt, mit Stickstoff entlüftet und in einen Zugabetrichter gegeben. Die Monomere, der Radikalstarter und das Kettentransfer-Reagenz wurden dann langsam während 3 Stunden in das Reaktionsgefäß gegeben.
Danach wurden erneut 0,1 g des Radikalstarters (Trigonox C) zugegeben. Nach weiteren 30 Minuten wurden nochmals 0,1 g des Radikalstarters (Trigonox C) zugegeben. Dies wurde nach weiteren 30 Minuten nochmals wiederholt, die Reaktion wiederum noch 30 Minuten fortgesetzt und das Reaktionsgemisch dann auf Raumtemperatur abgekühlt. Das Lösemittel wurde unter vermindertem Druck abdestilliert.

### Analyse des Produkts (SEC):

Mw = 10560
Polydispersität 3,64

### POLYMER 3

### Herstellung:

Lösemittel und Monomere wurden durch Stickstoffeinblasung entlüftet. Die Reaktion wurde unter Stickstoffatmosphäre durchgeführt.
Die Polymerisation wurde in einem 500 ml-Vierhalskolben durchgeführt, der mit einer Kühleinheit, eines - Heizmantel, Thermometer, mechanischem Rührer und einer Vorrichtung zur Stickstoffeinblasung ausgestattet war.
Das Lösemittel PMA (71,33 g) und das Monomer Silaplane FM0721 (26,38 g) wurden in das Reaktionsgefäß gegeben, gemischt, mit Stickstoff entlüftet und auf 110°C erwärmt.
Die weiteren Monomere (EHA (5,74 g), Makromonomer 2 (15,88 g)) sowie der Radikalstarter (Perkadox AMBN (0,30 g)) und das Kettentransfer-Reagenz (alpha-Methylstyrol-Dimer (0,37g)) wurden gemischt, mit Stickstoff entlüftet und in einen Zugabetrichter gegeben. Die Monomere, der Radikalstarter und das Kettentransfer-Reagenz wurden dann langsam während 3 Stunden in das Reaktionsgefäß gegeben. Nach beendeter Zugabe wurde die Reaktion noch für weitere 4,5 Stunden fortgesetzt. Danach wurden erneut 0,07 g des Radikalstarters zugegeben. Nach weiteren 30 Minuten wurden nochmals 0,07 g des Radikalstarters zugegeben. Dies wurde nach weiteren 30 Minuten nochmals wiederholt, die Reaktion danach noch 2 Stunden fortgesetzt und das Reaktionsgemisch dann auf Raumtemperatur abgekühlt. Das Lösemittel wurde unter vermindertem Druck abdestilliert.

### Analyse des Produkts (SEC):

Mw = 17410
Polydispersität 4,81

### POLYMER 4

### Herstellung:

Lösemittel und Monomere wurden durch Stickstoffeinblasung entlüftet. Die Reaktion wurde unter Stickstoffatmosphäre durchgeführt.
Die Polymerisation wurde in einem 500 ml-Vierhalskolben durchgeführt, der mit einer Kühleinheit, einem Heizmantel, Thermometer, mechanischem Rührer und einer Vorrichtung zur Stickstoffeinblasung ausgestattet war.
Das Lösemittel PMA (50,18g) wurde in das Reaktionsgefäß gegeben, gemischt, mit Stickstoff entlüftet und auf 110°C erwärmt.
Die Monomere (EHA (33,33 g), AA (0,77 g), Makromonomer 3 (43,9 g in PMA) sowie der Radikalstarter (Perkadox AMBN (0,82 g)) und das Kettentransfer-Reagenz (alpha-Methylstyrol-Dimer (1,01 g)) wurden gemischt, mit Stickstoff entlüftet und in einen Zugabetrichter gegeben. Die Monomere, der Radikalstarter und das Kettentransfer-Reagenz wurden dann langsam während 3 Stunden in das Reaktionsgefäß gegeben.
Nach beendeter Zugabe wurde die Reaktion noch für weitere 4 Stunden fortgesetzt. Danach wurden erneut 0,15 g des Radikalstarters zugegeben. Nach weiteren 30 Minuten wurden nochmals 0,15 g des Radikalstarters zugegeben. Dies wurde nach weiteren 30 Minuten nochmals wiederholt, die Reaktion danach noch 2 Stunden fortgesetzt und das Reaktionsgemisch dann auf Raumtemperatur abgekühlt. Das Lösemittel wurde unter vermindertem Druck abdestilliert.

### Analyse des Produkts (SEC):

Mw = 10508
Polydispersität 4,31

### POLYMER 5

### Herstellung:

Lösemittel und Monomere wurden durch Stickstoffeinblasung entlüftet. Die Reaktion wurde unter Stickstoffatmosphäre durchgeführt.
Die Polymerisation wurde in einem 500 ml-Vierhalskolben durchgeführt, der mit einer Kühleinheit, einem Heizmantel, Thermometer, mechanischem Rührer und einer Vorrichtung zur Stickstoffeinblasung ausgestattet war.
Das Lösemittel Isobutanol (85,24 g) wurde in das Reaktionsgefäß gegeben, gemischt, mit Stickstoff entlüftet und auf 110°C erwärmt.
Die Monomere (EHA (38,97 g), AA (1,86 g), BISOMER MPEG550MA (64,92 g), Makromonomer 3 (104,57 g) sowie der Radikalstarter (Perkadox AMBN (2 g)) und das Kettentransfer-Reagenz (alpha-Methylstyrol-Dimer (2,44 g)) wurden gemischt, mit Stickstoff entlüftet und in einen Zugabetrichter gegeben. Die Monomere, der Radikalstarter und das Kettentransfer-Reagenz wurden dann langsam während 4 Stunden in das Reaktionsgefäß gegeben.
Nach beendeter Zugabe wurde die Reaktion noch für weitere 3 Stunden fortgesetzt. Danach wurden erneut 0,15 g des Radikalstarters zugegeben. Nach weiteren 30 Minuten wurden nochmals 0,15 g des Radikalstarters zugegeben. Dies wurde nach weiteren 30 Minuten nochmals wiederholt, die Reaktion danach noch 2 Stunden fortgesetzt und das Reaktionsgemisch dann auf Raumtemperatur abgekühlt. Das Lösemittel wurde unter vermindertem Druck abdestilliert.

### Analyse des Produkts (SEC):

Mw = 10877
Polydispersität 2,81

### POLYMER 6

In einem 250-ml-4-Halskolben mit Rührer, Thermometer, Rückflußkühler und Tropftrichter werden bei Raumtemperatur 47,94 g Dowanol PMA und 2,57 g Silaplane FM-0721 vorgelegt. Während der gesamten Reaktion wird Stickstoff übergeleitet. Die Reaktionsmischung wird auf 135°C erwärmt. Nach Erreichen der Temperatur wird in 3 h die Mischung aus 43,96 g n-Butylacrylat, 4,88 g Makromonomer 4 und 0,5 g Trigonox C zudosiert. Nach Ende der Dosierung, und 30 min und 60 min später werden noch einmal je 0,25 g Trigonox C zugegeben. Nach weiteren 60 min wird der Ansatz beendet.

### Analyse des Produkts (SEC):

Mw = 15128
Polydispersität 3,14

### POLYMER 7

In einem 250-ml-4-Halskolben mit Rührer, Thermometer, Rückflußkühler und Tropftrichter werden bei Raumtemperatur 47,94 g Dowanol PMA und 2,57 g Silaplane FM-0721 vorgelegt. Während der gesamten Reaktion wird Stickstoff übergeleitet. Die Reaktionsmischung wird auf 135°C erwärmt. Nach Erreichen der Temperatur wird in 3 h die Mischung aus 39,07 g n-Butylacrylat, 9,77 g Makromonomer 4 und 0,5 g Trigonox C zudosiert. Nach Ende der Dosierung, und 30 min und 60 min später werden noch einmal je 0,25 g Trigonox C zugegeben. Nach weiteren 60 min wird der Ansatz beendet.

### Analyse des Produkts (SEC):

Mw = 22660
Polydispersität 4,73

### POLYMER 8

In einem 250-ml-4-Halskolben mit Rührer, Thermometer, Rückflußkühler und Tropftrichter werden bei Raumtemperatur 73,50 g Dowanol PMA und 4,45 g Silaplane FM-0721 vorgelegt. Während der gesamten Reaktion wird Stickstoff übergeleitet. Die Reaktionsmischung wird auf 135°C erwärmt. Nach Erreichen der Temperatur wird in 3 h die Mischung aus 19,91 g i-Butylmethacrylat, 13,05 g Styrol, 10,42 g HEMA, 1,16 g Makromonomer 4 und 0,67 g Trigonox C zudosiert. Nach Ende der Dosierung, und 30 min und 60 min später werden noch einmal je 0,112 g Trigonox C zugegeben. Nach weiteren 60 min wird der Ansatz beendet.

### Analyse des Produkts (SEC):

Mw = 19965
Polydispersität 3,99

### POLYMER 9

In einem 500-ml-4-Halskolben mit Rührer, Thermometer, Rückflußkühler und Tropftrichter werden bei Raumtemperatur 192 g Dowanol PMA vorgelegt. Während der gesamten Reaktion wird Stickstoff übergeleitet. Die Reaktionsmischung wird auf 135°C erwärmt. Nach Erreichen der Temperatur wird in 2,5 h die Mischung aus 176,80 g n-Butylacrylat, 31,20 g Makromonomer 4 und 1,03 g Trigonox C zudosiert. Nach Ende der Dosierung, und 30 min und 60 min später werden noch einmal je 0,52 g Trigonox C zugegeben. Nach weiteren 60 min wird der Ansatz beendet.

### Analyse des Produkts (SEC):

Mw = 14551
Polydispersität 2,96

### POLYMER 10

In einem 500-ml-4-Halskolben mit Rührer, Thermometer, Rückflußkühler und Tropftrichter werden bei Raumtemperatur 144 g Dowanol PMA vorgelegt. Während der gesamten Reaktion wird Stickstoff übergeleitet. Die Reaktionsmischung wird auf 135°C erwärmt. Nach Erreichen der Temperatur wird in 3 h die Mischung aus 107,48 g Ethylhexylacrylat, 25,12 g Ethylacrylat, 32,96 g Makromonomer 4 und 0,773 g Trigonox C zudosiert. Nach Ende der Dosierung, und 30 min und 60 min später werden noch einmal je 0,38 g Trigonox C zugegeben. Nach weiteren 60 min wird der Ansatz beendet.

### Analyse des Produkts (SEC):

Mw = 8798
Polydispersität 2,26

### POLYMER 11

PMA (47,4 g), Makromonomer 3 (50 g) und alpha-Methylstyrol-Dimer (1,1 g) werden in einem Reaktionsgefäß unter Stickstoff auf 120°C erwärmt.
Nun wird eine Mischung aus DMAEMA (15,2 g) und Perkadox AMBN (1,1 g) bei 0,5 ml/min hinzudosiert. Danach wird Perkadox AMBN (0,5 g) zur Reaktion addiert. Die Nachreaktionszeit beträgt 2 Stunden. Die Polymerlösung wird mit PMA auf einen Festkörper von 40% eingestellt.
Mn = 6751 g/mol
Mw/Mn = 5,42

### Vergleichsbeispiel:

### Vergleichs-Polymer V1

### (statistisches Copolymer hergestellt mit linearem MPEG-Monomethacrylat (Blemmer PME 1000)

PMA (56,5 g), Blemmer PME 1000 (38 g) und alpha-Methylstyrol-Dimer (1,1 g) werden in einem Reaktionsgefäß unter Stickstoff auf 120°C erwärmt.
Nun wird eine Mischung aus DMAEMA (16,3 g) und Perkadox AMBN (1,1 g) bei 0,5 ml/min hinzudosiert. Danach wird Perkadox AMBN (0,5 g) zur Reaktion addiert. Die Nachreaktionszeit beträgt 2 Stunden. Die Polymerlösung weist einen Festkörper von 50% auf.
Mn = 10125 g/mol
Mw/Mn = 5,67

### POLYMER 12 (Dispergiermittel 3)

PMA (47,4 g), Makromonomer 3 (50 g) und alpha-Methylstyrol-Dimer (1,1 g) werden in einem Reaktionsgefäß unter einer Stickstoffatmosphäre auf 120°C erwärmt.
Nun wird eine Mischung aus DMAEMA (15,2 g) und Perkadox AMBN (1,1 g) bei 0,5 ml/min hinzudosiert. Danach wird Perkadox AMBN (0,5 g) zur Reaktion addiert.
Es werden 12 g Benzylchlorid und 30 g Butylglycol hinzugegeben und zwei Stunden bei 120°C zur Reaktion gebracht. Die Polymerlösung wird mit PMA auf einen Festkörper von 40% eingestellt.

### POLYMER 13 (Dispergiermittel 4)

PMA (47,4 g), Makromonomer 3 (50 g) und alpha-Methylstyrol-Dimer (1,1 g) werden in einem Reaktionsgefäß unter einer Stickstoffatmosphäre auf 120°C erwärmt.
Nun wird eine Mischung aus DMAEMA (15,2 g) und Perkadox AMBN (1,1 g) bei 0,5 ml/min hinzudosiert. Danach wird Perkadox AMBN (0,5 g) zur Reaktion addiert.
Es werden 25 g Disperbyk 102 hinzugegeben und 30 min bei 50°C homogenisiert. Die Polymerlösung wird mit PMA auf einen Festkörper von 40% eingestellt.

### POLYMER 14 (Dispergiermittel 5)

PMA (47,4 g), Makromonomer 3 (50 g) und alpha-Methylstyrol-Dimer (1,1 g) werden in einem Reaktionsgefäß unter einer Stickstoffatmosphäre auf 120°C erwärmt.
Nun wird eine Mischung aus Vinylimidazol (15,2 g) und Perkadox AMBN (1,1 g) bei 0,5 ml/min hinzudosiert. Danach wird Perkadox AMBN (0,5 g) zur Reaktion addiert. Die Nachreaktionszeit beträgt 2 Stunden. Die Polymerlösung wird mit PMA auf einen Festkörper von 40% eingestellt.

### ANWENDUNGSBEISPIELE

### a) Verlaufmittel

Prüfsystem: Acrylat-Melamin-Einbrennlack, klar

### Zusammensetzung (Angaben in Gewichtsteilen)

| | |
|---|---|
| Setalux 1760 VB 64 | 44,0 |
| Setalux C 91389 VX 45 | 16,5 |
| Luwipal 018 | 25,3 |
| Butyldiglykolacetat | 2,1 |
| Solvesso 150 | 4,8 |
| Butanol | 6,1 |
| Butyldiglykol | 1,2 |

mischen, dann Zugabe von

| | |
|---|---|
| Butanol | 8,0 |
| Solvesso 150 | 3,8 |
| Butyldiglykol | 1,8 |

- Setalux 1760 VB 64: = Acrylat-Einbrennharz Nuplex Resins, Bergen op Zoom
- Setalux C 91389 VX 45: = Acrylat-Einbrennharz Nuplex Resins, Bergen op Zoom
- Luwipal 018: = Melamin-Einbrennharz, BASF AG, Ludwigshafen

Die Verlaufmittel (Polymer 6, Polymer 7, Polymer 9, Polymer 10, Modaflow oder kein Verlaufmittel, gemäß untenstehender Tabelle) wurden einen Tag vor der Applikation eingearbeitet. Die Dosierung betrug 0,15 Gew.%, bezogen auf das Gesamtgemisch. Am Tag der Applikation wurde mit Solvesso 150 die Viskosität auf 24 Sekunden, DIN 4mm-Auslaufbecher (23°C) eingestellt. Die Applikation erfolgte mittels Spritzautomaten.
Die Aushärtung erfolgte nach 30 Minuten Ablüftzeit bei 140°C in 30 Minuten.
Die Schichtdicke betrug 20 µm.
Der Verlauf wurde mit dem wave-scan DOI der Firma Byk-Gardner ermittelt, indem die Lange Welle, die Kurze Welle und der Wert für DOI (distinctness of image) gemessen wurden. Glanz und Haze wurden mit dem Haze-Gloss der Firma Gardner ermittelt.

| | Lange Welle | Kurze Welle | DOI | Glanz R20 | Haze |
|---|---|---|---|---|---|
| Ohne Add. | 58 | 64 | 73 | 89 | 76 |
| Modaflow* | 18 | 23 | 90 | 94 | 18 |
| Polymer 6 | 2 | 14 | 92 | 97 | 13 |
| Polymer 7 | 17 | 20 | 92 | 97 | 14 |
| Polymer 9 | 12 | 16 | 92 | 97 | 14 |
| Polymer 10 | 11 | 14 | 92 | 97 | 13 |

| | | | | | |
|---|---|---|---|---|---|
| *MODAFLOW ist ein Verlaufmittel der Cytec Industries | | | | | |

Die Ergebnisse zeigen, dass bei Verwendung der Polymere als Verlaufmittel bessere Ergebnisse hinsichtlich Verlauf, Glanz und Haze erzielt werden als bei Verwendung eines herkömmlichen Verlaufmittels oder ohne Verlaufmittel.

### b) Schmutzabweisende Oberflächen

Prüfsystem: Acrylat/Melamin-Einbrennlack:

| | |
|---|---|
| Setalux C1502 | 54,00 % |
| Maprenal MF600 | 28,00% |
| Shellsol A | 4,00% |
| Solvesso 150 | 4,00% |
| Xylol | 10,00% |
| Total | 100,00% |

- Setalux C1502: = Acrylat-Einbrennharz, Nuplex Resins, NL-Bergen op Zoom
- Maprenal MF600: = Melamin-Einbrennharz, Ineos Melamines, D-Frankfurt

Bei den Prozentangaben handelt es sich um Gewichtsprozent, bezogen auf das Gesamtgemisch.

### Herstellung des Acrylat-Melamin-Einbrennlackes :

Alle Komponenten werden gemischt und 10 Minuten mit einem Dissolver bei einer Umfangsgeschwindigkeit von 5 m/s homogenisiert. Danach wird die Viskosität mit Xylol auf 24 Sekunden, DIN 4mm-Becher (23°C), eingestellt.
Die zu prüfenden Additive werden 10 Minuten mit einem Scandex-Rüttler in einer Konzentration von 1 Gew.-% Wirksubstanz, bezogen auf das Gesamtgemisch, in den Lack eingearbeitet.
Nach der Einarbeitung werden die additivierten Lacke in einem 100 µm-Nassfilm mit einem Spiralrakel auf ein geprimertes Alublech aufgezogen. Nach einer Ablüftzeit von 10 Minuten werden die Bleche 20 Minuten bei 140°C eingebrannt. Von jedem additivierten Lack werden jeweils zwei Bleche gefertigt.

Die erhaltenen Lackfilme werden hinsichtlich ihrer schmutz-, wasser- und ölabweisenden Wirkung nach folgenden Kriterien beurteilt:

### Visuelle Beurteilung der Lackoberfläche:

Die Lackoberfläche wird auf Trübung im Film und Oberflächenstörungen, wie Krater, Stippen und Unverträglichkeiten, untersucht.

### Bewertung: 1 - 5

- 1 =: Lackoberfläche ohne Oberflächenstörungen
- 5 =: Lackoberfläche mit vielen Oberflächenstörungen

### Edding-Test:

Es wird mit einem Permanent-Marker des Typs "Edding 400" die Lackoberfläche beschriftet und visuell beurteilt, ob die Oberfläche beschreibbar ist. Es wird beurteilt, ob die Tinte auf der Oberfläche spreitet oder sich zusammenzieht. Nach dem Trocknen der Tinte wird versucht, diese mit einem trockenen Tuch abzuwischen.

### Bewertung: 1 - 5

- 1 =: Tinte zieht sich zusammen, läßt sich restlos mit einem Papiertuch entfernen
- 5 =: Tinte spreitet sehr gut auf Untergrund, läßt sich praktisch nicht entfernen

### Bitumen-Test:

Bitumen wird erhitzt, bis er so weit verflüssigt ist, dass er sich auf die Lackoberfläche aufbringen lässt. Nach Abkühlen der Masse wird visuell beurteilt, wie gut sich die Bitumenmasse von der Oberfläche wieder rückstandsfrei manuell ablösen lässt.

### Bewertung: 1 - 5:

- 1 =: Bitumen läßt sich leicht und restlos entfernen
- 5 =: Bitumen haftet fest auf der Oberfläche und läßt sich praktisch nicht entfernen

### Verschmutzung mit Bayferrox-Pulver:

Auf die Lackoberfläche werden 3 Löffel Bayferrox 130M, Eisenoxid-Pigment, Bayer AG, gestreut und mit dest. Wasser in 5 Spritzgängen mit Hilfe einer Spritzflasche wieder abgespült. Die möglichst rückstandsfreie Oberfläche wird visuell beurteilt.

### Bewertung: 1 - 5:

- 1 =: Bayferrox-Pulver läßt sich rückstandslos mit Wasser abwaschen
- 5 =: kein Reinigungseffekt beim Abspülen mit Wasser, ein großer roter Fleck verbleibt

### Wasser-Ablauftest:

Es wird ein Tropfen Wasser auf die Oberfläche gegeben. Anschließend wird die beschichtete Lackoberfläche gekippt, bis der Tropfen abläuft. Es wird visuell beurteilt, bei welchem Winkel der Tropfen abläuft und ob der Tropfen rückstandsfrei abläuft.

### Bewertung: 1 - 5:

- 1 =: geringer Winkel genügt und der Tropfen läuft komplett ohne Nasenbildung und Rückstandströpfchen ab
- 5 =: Lackblech muss stark geneigt werden bis der Tropfen abläuft, eventuell bleiben Wasserreste auf der Lackoberfläche zurück

### Mineralöl-Ablauftest:

Es wird ein Tropfen handelsübliches Mineralöl auf die Lackoberfläche gegeben. Anschließend wird die beschichtete Lackoberfläche gekippt, bis der Tropfen etwa 10 cm gelaufen ist. Nach Ablauf von 5 Minuten wird die Ölspur bzw. eine erneute Tropfenbildung visuell bewertet.

### Bewertung: 1 - 5:

- 1 =: Die Ölspur bildet sich sofort in einzelne Tropfen zurück
- 5 =: Die Ölspur bildet sich nicht zurück, sondern spreitet eventuell weiter

Die erhaltenen Ergebnisse sind in der folgenden Tabelle dargestellt.

| | Oberfläche | Edding | Bitumen | Bayferrox | Wasserab. | Mineralöl |
|---|---|---|---|---|---|---|
| Kontrolle ohne Additiv | 2 | 5 | 5 | 5 | 5 | 5 |
| Polymer 3 | 1 | 1 | 1 | 1 | 1 | 1 |
| Polymer 8 | 1 | 1 | 1 | 1 | 1 | 1 |

Die Ergebnisse zeigen, dass durch die Verwendung der Polymere als Additive eine sehr gut schmutz-, öl- und wasserabweisende Oberfläche erzielt wird.

### c) Netz- und Dispergiermittel

Verwendung der Polymere als Netz- und Dispergiermittel zur Herstellung von Pigmentkonzentraten und deren Einsatz in Lacksystemen

### Ausgangsstoffe

| | |
|---|---|
| Uralac SN 831 | Polyesterharz, Hersteller DSM Resins |
| Cymel 303 | Melamin-Formaldehyd-Harz, Hersteller Cytec Industries |
| Dynapol Catalyst 1203 | Katalysator, Hersteller Evonik Degussa |
| Ti Pure R960 | Titandioxid-Pigment, Hersteller Du Pont |
| Bayferrox 140M | Eisenoxidrotpigment, Hersteller Lanxess |
| Aerosil R972 | Hydrophobe, pyrogene Kieselsäure, Hersteller Degussa |
| BYK 057 | Silikonfreier Polymer-Entschäumer, Hersteller Byk Chemie |
| BYK 355 | Acrylatverlaufadditiv, Hersteller Byk Chemie |
| Solvesso 150 ND | Aromatisches Lösemittel, Hersteller ExxonMobil |
| Dowanol PMA | 1-Methoxy-2-propylacetat, Hersteller Dow Chemical |

### Arbeitsverfahren

Herstellung der Ti Pure R960-Pigmentkonzentrate

### Mahlbedingungen:

Gerät: Lau Paint Shaker DAS H [/A]200-K
Mahlzeit: 60 min, normale Geschwindigkeit, maximale Kühlung
Mahlgutzusatz: 50 Gew.-% Glasperlen (1 mm Durchmesser)

Zusammensetzung der Ti Pure 960 - Pigmentkonzentrate:

| | erfindungsgemäß: Pigmentkonzentrat Ti pure 1 | Vergleichsbeispiel: Pigmentkonzentrat Ti pure 2 |
|---|---|---|
| Mahlgut: | | |
| Uralac SN831 | 26.2 | 26.2 |
| Additiv Polymer 11 | 2.1 | - |
| Additiv Vergleichs-Polymer V1 | - | 2.1 |
| Dowanol PMA | 5.0 | 5.0 |
| Solvesso 150ND | 10.8 | 11.2 |
| Aerosil R972 | 0.6 | 0.6 |
| BYK-057 | J.3 | 0.3 |
| Ti pure R960 | 55.0 | 55.0 |
| | 100.0 | 100.0 |

### Herstellung der Bayferrox 140M - Pigmentkonzentrate:

### Mahlbedingungen:

Gerät: Lau Paint Shaker DAS H [/A]200-K
Mahlzeit: 120 min, normale Geschwindigkeit, maximale Kühlung
Mahlgutzusatz: 50 Gew.-% Glasperlen (1 mm Durchmesser)

Zusammensetzung der Bayferrox 140M-Pigmentkonzentrate:

| | erfindungsgemäß: Pigmentkonzentrat Bayferrox 1 | Vergleichsbeispiel: Pigmentkonzentrat Bayferrox 2 |
|---|---|---|
| Mill base: | | |
| Uralac SN831 | 33.7 | 33.7 |
| Additiv Polymer 11 | 8.4 | - |
| Additiv Vergleichs-Polymer V1 | - | 3.4 |
| Dowanol PMA | 5.0 | 5.0 |
| Solvesso 150ND | 7.1 | 8.7 |
| Aerosil R972 | 0.5 | 0.5 |
| BYK-057 | 0.3 | 0.3 |
| Bayferrox 140M | 45.0 | 45.0 |
| | 100.0 | 100.0 |

Herstellung der Testformulierungen

### Zusammensetzung des Klarlacks

| *Klarlack* | |
|---|---|
| Uralac SN 831 | 64.0 |
| Cymel 303 | 13.8 |
| Dynapol Catalyst 1203 | 4.9 |
| BYK-057 | 0.4 |
| BYK-355 | 1.1 |
| Solvesso 150 ND | 10.5 |
| Dowanol PMA | 5.3 |
| | 100.0 |

### Zusammensetzung der Testformulierungen

| | Weiß : rot 70:30 |
|---|---|
| Klarlack | 21.9 |
| Pigmentkonzentrat Ti Pure R960 | 19.1 |
| Pigmentkonzentrat Bayferrox 140M | 9.0 |
| | 50.0 |

Es wurde eine Testformulierung hergestellt, welche die mit Polymer 11 hergestellten Pigmentkonzentrate enthielt (Pigmentkonzentrat Ti pure 1 und Pigmentkonzentrat Bayferrox 1), sowie eine Vergleichs-Testformulierung, welche die mit dem Polymer des Vergleichsbeispiels V1 hergestellten Pigmentkonzentrate enthielt (Pigmentkonzentrat Ti pure 2 und Pigmentkonzentrat Bayferrox 2).

Die Viskosität der Testformulierungen wurde im Anschluss an die Herstellung mit Solvesso 150ND auf 90 - 110 sec (DIN 4 - Auslaufbecher, 23°C) eingestellt. Die hierzu jeweils benötigte Menge Solvesso 150ND läßt sich der folgenden Tabelle entnehmen:

| Testformulierung mit.... | Anfangsviskosität [s] | Erforderliche Menge Solvesso 150ND | Endviskosität [s] |
|---|---|---|---|
| Pigmentkonzentraten Ti pure 1 und Bayferrox 1 (erfindungsgemäß) | 99 | - | 99 |
| Pigmentkonzentraten Ti pure 2 und Bayferrox 2 (Vergleichsbeispiel) | 120 | 1 % | 105 |

### Ergebnis:

Durch die Verwendung des Polymers 11 (hergestellt mit erfindungsgemäßen verzweigten oxetan-basierten Makromonomeren) als Netz- und Dispergiermittel wird ein Beschichtungsmittel mit niedrigerer Viskosität erhalten als die Vergleichsformulierung mit dem nicht erfindungsgemäßen Vergleichspolymer V1 (hergestellt mit nicht erfindungsgemäßen linearen Polyether-Monomeren).

### Applikation der Testformulierung

Die Testformulierungen wurden am Tag der Herstellung unter den folgenden Bedingungen auf Substrate aufgetragen.

| Substrate: | Alcan Aluminiumbleche, vorbeschichtet mit einer etwa 5 µm dicken PU-Primerschicht |
|---|---|
| Rakelauftrag: | 80 µm (naß) |
| Ofentemperatur: | 320°C |
| Einbrennzeit: | 30 s |
| max. Metallltemperatur: | 235°C |
| Trockenfilmschichtdicke: | 18-20 µm |

Die Ausschwimm-, Aufschwimm- und Flokkulationseigenschaften wurden wie folgt evaluiert.

### Rub-Out-Test

Unmittelbar nach der Applikation wurden die frisch aufgetragenen Lackschichten auf beiden Hälften der Substratoberfläche einem Rub-Out-Test unterzogen.
Hierzu wurden einige Stellen der frischen Lackschichten nach dem Auftrag mechanisch verrieben und anschließend die durch das Verreiben hervorgerufene Farbtonänderung (Rub-Out-Effekt) gegenüber dem nicht verriebenen Lack gemessen. Ursachen für den Rub-Out-Effekt sind z.B. Flokkulation und/oder Aufschwimmen von Pigmenten. Falls Pigmentflokkulate vorliegen, werden diese durch die Scherwirkung beim Reiben zerstört, und es entsteht der ursprünglich angestrebte Farbton. Der Rub-Out-Effekt läßt sich durch geeignete Dispergiermittel minimieren und ist somit ein Maß für die Wirksamkeit eines Dispergiermittels.

### Farbmetrische Messungen (ΔE, Lab)

| | |
|---|---|
| Gerät: | Color guide, BYK-Gardner |
| Lichtart: | Normlicht D65 (Tageslicht) |
| Meßfeld: | 10° |
| Meßgeometrie: | d/8° spin (diffuse Beleuchtung, Beobachtungswinkel 8°) |
| Anzahl der Messungen: | n = 3 |

Die Ergebnisse der Farbmetrik sind in der folgenden Tabelle wiedergegeben.
Der für eine Formulierung ermittelte ΔE-Wert gibt den Farbunterschied zwischen den verriebenen und den nicht verriebenen Stellen der Beschichtung an (Rub-Out-Effekt). Die Lab-Werte entsprechen den Farbmaßzahlen L (Helligkeit), a (rot-grün) und b (blau-gelb) des CIELAB-Farbraums.

| Testformulierung mit... | ΔE nicht gerührt | Lab nicht gerührt | Glanz 60° |
|---|---|---|---|
| Pigmentkonzentraten | | 50,73 | |
| Ti pure 1 und | 0,13 | 21,02 | 93 |
| Bayferrox 1 (erfindungsgemäß) | | 6,95 | |
| Pigmentkonzentraten | | 50,77 | |
| Ti pure 2 und | 0,47 | 20,39 | 92 |
| Bayferrox 2 (Vergleichsbeispiel) | | 6,25 | |

Durch die Verwendung der mit erfindungsgemäßen oxetan-basierten Makromonomeren erhaltenen Polymere als Netz- und Dispergiermittel werden Formulierungen erhalten, die auch ohne vorheriges Aufrühren einen niedrigeren Rub-Out-Effekt (AE-Wert) als solche Formulierungen aufweisen, die herkömmliche, mit linearen Polyether-basierten Monomeren hergestellte Dispergiermittel enthalten.

## Patentansprüche

1. Verfahren zur Herstellung eines Makromonomers, **dadurch gekennzeichnet, dass** mindestens ein Hydroxyoxetan der Formel (I) 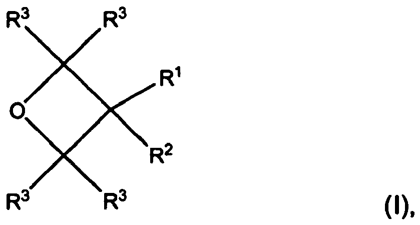 worin
R¹ für Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
R² für Hydroxyalkyl, Hydroxyalkoxy, Hydroxyalkoxyalkyl, Hydroxyaryl oder Hydroxyaryloxy steht, und
R³ unabhängig voneinander jeweils für Wasserstoff, Alkyl, Alkoxy, Alkoxyalkyl, Aryloxyalkyl, Hydroxyalkyl, Hydroxyalkoxy, Hydroxyalkoxyalkyl, Aryl, Aryloxy, Hydroxyaryl oder Hydroxyaryloxy steht,
mit einem Startmolekül U - L - X - H, worin
U = eine von Allyl [H₂C = CH - CH₂ -] verschiedene, ethylenisch ungesättigte, radikalisch polymerisierbare Kopfgruppe,
L = zweibindiger organischer Rest ohne radikalisch polymerisierbare Gruppen, und X -H = nicht radikalisch polymerisierbare Gruppe mit aktivem Wasserstoffatom H ist,
in einem molaren Verhältnis (Hydroxyoxetan(e) der Formel (I) : Startmolekül) von (100 : 1) bis (1 : 1) in Anwesenheit mindestens eines geeigneten Katalysators in einer kationischen ringöffnenden Polymerisation umgesetzt wird.

2. Verfahren nach Anspruch 1, **dadurch gekennzeichnet, dass** die Umsetzung in Anwesenheit mindestens eines Inhibitors der radikalischen Polymerisation erfolgt.

3. Verfahren nach einem der Ansprüche 1 oder 2, **dadurch gekennzeichnet, dass** das molare Verhältnis (Hydroxyoxetan(e) der Formel (1) : Startmolekül) von (50 : 1) bis (2 : 1), bevorzugt von (25 : 1) bis (3 : 1) beträgt.

4. Verfahren nach einem der vorhergehenden Ansprüche, **dadurch gekennzeichnet, dass** das mindestens eine Hydroxyoxetan der Formel (I)
ein 3-Alkyl-3-(hydroxyalkyl)oxetan [d.h. R1 = Alkyl, R2 = Hydroxyalkyl, alle R3 = H],
ein 3,3-Di(hydroxyalkyl)oxetan [d.h. R1, R2 = Hydroxyalkyl, alle R3 = H],
ein 3-Alkyl-3-(hydroxyalkoxy)oxetan [d.h. R1 = Alkyl, R2 = Hydroxyalkoxy, alle R3 = H],
ein 3-Alkyl-3-(hydroxyalkoxyalkyl)oxetan [d.h. R1 = Alkyl, R2 = Hydroxyalkoxyalkyl, alle R3 = H],
oder ein Gemisch dieser Hydroxyoxetane,
bevorzugt ein 3-Alkyl-3-(hydroxyalkyl)oxetan [d.h. R1 = Alkyl, R2 = Hydroxyalkyl, alle R3 = H] und/oder ein 3-Alkyl-3-(hydroxyalkoxy)oxetan [d.h. R¹ = Alkyl, R² = Hydroxyalkoxy, alle R³ = H],
ist.

5. Verfahren nach einem der vorhergehenden Ansprüche, **dadurch gekennzeichnet, dass** das mindestens eine Hydroxyoxetan
3-Methyl-3-(hydroxymethyl)oxetan [d.h. R1 = Methyl, R2 = Hydroxymethyl, alle R3 = H],
3-Ethyl-3-(hydroxymethyl)oxetan [d.h. R1 = Ethyl, R2 = Hydroxymethyl, alle R3 = H] (= Trimethylolpropanoxetan),
an der Hydroxygruppe ethoxylierte und/oder propoxylierte Derivate des Trimethylolpropanoxetans mit 1 - 20 Ethylenoxid- und/oder Propylenoxid-Einheiten, die statistisch, blockweise oder gradientenartig angeordnet sein können,
3,3-Di(hydroxymethyl)oxetan [d.h. R1 = R2 = Hydroxymethyl, alle R3 = H],
oder ein Gemisch dieser Hydroxyoxetane,
bevorzugt 3-Ethyl-3-(hydroxymethyl)oxetan (= Trimethylolpropanoxetan) und/oder mindestens ein an der Hydroxygruppe ethoxyliertes und/oder propoxyliertes Derivat des Trimethylolpropanoxetans mit 1 - 20 Ethylenoxid- und/oder Propylenoxid-Einheiten, die statistisch, blockweise oder gradientenartig angeordnet sein können, ist.

6. Verfahren nach einem der vorhergehenden Ansprüche, **dadurch gekennzeichnet, dass** die ethylenisch ungesättigte Kopfgruppe U = Acryloyl [H₂C = CH - C(=O)-] oder Methacryloyl [H₂C = C(CH₃) - C(=O)-] ist.

7. Verfahren nach einem der vorhergehenden Ansprüche, **dadurch gekennzeichnet, dass** der zweibindige organische Rest L die Struktur -O-L' aufweist, wobei -O- ein Sauerstoffatom ist und L' aus der Gruppe bestehend aus Alkylenresten und Polyalkylenoxidresten ausgewählt wird.

8. Verfahren nach einem der vorhergehenden Ansprüche, **dadurch gekennzeichnet, dass** die Gruppe X - H eine Hydroxygruppe O - H oder eine Thiolgruppe S - H, bevorzugt eine Hydroxygruppe O - H ist.

9. Verfahren nach einem der vorhergehenden Ansprüche, **dadurch gekennzeichnet, dass** das Startmolekül U - L - X - H aus der Gruppe, bestehend aus Hydroxyalkyl-Estern der Methacrylsäure [H₂C = C(CH₃) - C(=O) - O - (CH₂)ₐ - OH] und Polyethylenöxid-Estern der Methacrylsäure [H₂C = C(CH₃) - C(=O) - O - (CH₂ - CH₂ - O)ₐ - H] mit a = 1 - 20, bevorzugt a = 1 - 10 und besonders bevorzugt a = 2 - 5, ausgewählt ist.

10. Verfahren nach einem der vorhergehenden Ansprüche, **dadurch gekennzeichnet, dass** das Startmolekül U - L - X - H Hydroxyethylmethacrylat oder Hydroxybutylmethacrylat ist.

11. Makromonomer, erhältlich nach dem Verfahren aus einem der Ansprüche 1 - 10.

12. Verwendung eines Makromonomers nach Anspruch 11 zur Herstellung eines Polymers durch radikalische Polymerisation.

## Claims

1. A process for preparing a macromonomer, **characterized in that** at least one hydroxyoxetane of the formula (I) 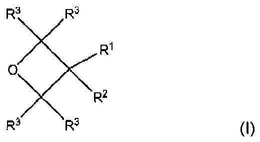 In which
R¹ is alkyl, alkoxy, alkoxyalkyl, aryloxyalkyl, hydroxyalkyl, hydroxyalkoxy, hydroxyalkoxyalkyl, aryl, aryloxy, hydroxyaryl or hydroxyaryloxy,
R² is hydroxyalkyl, hydroxyalkoxy, hydroxyalkoxyalkyl, hydroxyaryl or hydroxyaryloxy, and
R³ is in each case independently hydrogen, alkyl, alkoxy, alkoxyalkyl, aryloxyalkyl, hydroxyalkyl, hydroxyalkoxy, hydroxyalkoxyalkyl, aryl, aryloxy, hydroxyaryl or hydroxyaryloxy,
is reacted with a starter molecule U - L - X - H in which
U = an ethylenically unsaturated, free-radically polymerizable head group other than allyl [H₂C = CH - CH₂ -],
L = divalent organic radical without free-radically polymerizable groups, and
X -H = group which cannot be polymerized free-radically and has an active hydrogen atom H,
In a molar ratio (hydroxyoxetane(s) of the formula (I): starter molecule) of (100 : 1) to (1 : 1) in the presence of at least one suitable catalyst in a cationic ring-opening polymerization.

2. The process as claimed in claim 1, **characterized in that** the reaction is effected in the presence of at least one inhibitor of free-radical polymerization.

3. The process as claimed in either of claims 1 and 2, **characterized in that** the molar ratio (hydroxyoxetane(s) of the formula (I): starter molecule) is from (50 : 1) to (2 : 1), preferably from (25 : 1) to (3 : 1).

4. The process as claimed in any of the preceding claims, **characterized in that** the at least one hydroxyoxetane of the formula (I) is
a 3-alkyl-3-(hydroxyalkyl)oxetane [i.e. R¹ = alkyl, R² = hydroxyalkyl, all R³ = H],
a 3,3-di(hydroxyalkyl)oxetane [i.e. R¹, R² = hydroxyalkyl, all R³ = H],
a 3-alkyl-3-(hydroxyalkoxy)oxetane [i.e. R¹ = alkyl, R² = hydroxyalkoxy, all R³ = H],
a 3-alkyl-3-(hydroxyalkoxyalkyl)oxetane [i.e. R¹ = alkyl, R² = hydroxyalkoxyalkyl, all R³ = H],
or a mixture of these hydroxyoxetanes,
preferably a 3-alkyl-3-(hydroxyalkyl)oxetane [i.e. R¹ = alkyl, R² = hydroxyalkyl, all R³ = H] and/or a 3-alkyl-3-(hydroxyalkoxy)oxetane [i.e. R¹ = alkyl, R² = hydroxyalkoxy, all R³ = H].

5. The process as claimed in any of the preceding claims, **characterized in that** the at least one hydroxyoxetane is
3-methyl-3-(hydroxymethyl)oxetane [i.e. R¹ = methyl, R² = hydroxymethyl, all R³ = H],
3-ethyl-3-(hydroxymethyl)oxetane [i.e. R¹ = ethyl, R² = hydroxymethyl, all R³ = H] (= trimethylolpropane oxetane),
derivatives of trimethylolpropane oxetane which have been ethoxylated and/or propoxylated on the hydroxyl group and have 1 -20 ethylene oxide and/or propylene oxide units which may be arranged randomly, in blocks or in the manner of a gradient,
3,3-di(hydroxymethyl)oxetane [i.e. R¹ = R² = hydroxymethyl, all R³ = H],
or a mixture of these hydroxyoxetanes,
preferably 3-ethyl-3-(hydroxymethyl)oxetane (= trimethylolpropane oxetane) and/or at least one derivative of trimethylolpropane oxetane which has been ethoxylated and/or propoxylated on the hydroxyl group and has 1 - 20 ethylene oxide and/or propylene oxide units which may be arranged randomly, in blocks or in the manner of a gradient.

6. The process as claimed in any of the preceding claims, **characterized in that** the ethylenically unsaturated head group is U = acryloyl [H₂C = CH - C(=O)-] or methacryloyl [H₂C = C(CH₃) - C(=O)-].

7. The process as claimed in any of the preceding claims, **characterized in that** the divalent organic L radical has the structure -O-L' where -O- is an oxygen atom and L' is selected from the group consisting of alkylene radicals and polyalkylene oxide radicals.

8. The process as claimed in any of the preceding claims, **characterized in that** the X- H group is a hydroxyl group O - H or a thiol group S - H, preferably a hydroxyl group O - H.

9. The process as claimed in any of the preceding claims, **characterized in that** the starter molecule U - L - X - H is selected from the group consisting of hydroxyalkyl esters of methacrylic acid [H₂C = C(CH₃) - C(=O) - O - (CH₂)ₐ - OH] and polyethylene oxide esters of methacrylic acid [H₂C = C(CH₃) - C(=O) - O - (CH₂ - CH₂ - O)ₐ - H] where a = 1 - 20, preferably a = 1 - 10 and more preferably a = 2 - 5.

10. The process as claimed in any of the preceding claims, **characterized In that** the starter molecule U - L - X - H is hydroxyethyl methacrylate or hydroxybutyl methacrylate.

11. A macromonomer obtainable by the process from any of claims 1 - 10.

12. The use of a macromonomer as claimed in claim 11 for preparation of a polymer by free-radical polymerization.

## Revendications

1. Procédé pour la fabrication d'un macromonomère, **caractérisé en ce qu'**au moins un hydroxyoxétane de formule (I) 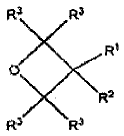 où
R¹ représente alkyle, alcoxy, alcoxyalkyle, aryloxyalkyle, hydroxyalkyle, hydroxyalcoxy, hydroxyalcoxyalkyle, aryle, aryloxy, hydroxyaryle ou hydroxyaryloxy,
R² représente hydroxyalkyle, hydroxyalcoxy, hydroxyalcoxyalkyle, hydroxyaryle ou hydroxyaryloxy, et
R³ respectivement représente, indépendamment les uns des autres, hydrogène, alkyle, alcoxy, alcoxyalkyle, aryloxyalkyle, hydroxyalkyle, hydroxyalcoxy, hydroxyalcoxyalkyl, aryle, aryloxy, hydroxyaryle ou hydroxyaryloxy,
avec une molécule de départ U - L - X - H, où
U = un groupe de tête éthyléniquement non saturé, polymerisable par radicaux et différent d'allyle [H₂C = CH - CH₂ -],
L = un groupement organique à deux liaisons sans groupes polymerisables par radicaux, et
X-H = un groupe non polymerisable par radicaux, avec un atome d'hydrogène H actif,
dans un rapport molaire (hydroxyoxétane(s) de formule (I) : molécule de départ) de (100 : 1) à (1 : 1) par polymérisation cationique par ouverture de cycle en présence d'au moins un catalyseur approprié.

2. Procédé selon la revendication 1, **caractérisé en ce que** ladite réaction s'effectue en présence d'au moins un inhibiteur de la polymérisation par radicaux.

3. Procédé selon l'une quelconque des revendications 1 ou 2, **caractérisé en ce que** le rapport molaire (hydroxyoxétane(s) de formule (I) : molécule de départ) est compris entre (50 : 1) à (2 : 1), de préférence entre (25 : 1) à (3 : 1).

4. Procédé selon l'une quelconque des revendications précédentes, **caractérisé en ce que** le au moins un hydroxyoxétane de formule (I) est
un 3-alkyle-3-(hydroxyalkyle)oxétane [c'est-à-dire, R1 = alkyle, R2 = hydroxyalkyle, tous les R3 = H],
un 3,3-dl(hydroxyalkyle)oxétane [c'est-à-dire, R1, R2 = hydroxyalkyle, tous les R3 = H],
un 3-alkyle-3-(hydroxyalcoxy)oxétane [c'est-à-dire, R1 = alkyle, R2 = hydroxyalcoxy, tous les R3 = H],
un 3-alkyle-3-(hydroxyalcoxyalkyle)oxétane [c'est-à-dire, R1 = alkyle, R2 = hydroxyalcoxyalkyle, tous les R3 = H],
ou un mélange de ces hydroxyoxétanes,
de préférence un 3-alkyle-3-(hydroxyalkyle)oxétane [c'est-à-dire, R1 = alkyle, R2 = hydroxyalkyle, tous les R3 = H], et/ou un 3-alkyle-3-(hydroxyalcoxy)oxétane [c'est-à-dire, R¹ = alkyle, R² = hydroxyalcoxy, tous les R³ = H].

5. Procédé selon l'une quelconque des revendications précédentes, **caractérisé en ce que** le au moins un hydroxyoxétane de formule (I) est
3-méthyle-3-(hydroxyméthyle)oxétane [c'est-à-dire, R1 = méthyle, R2 = hydroxyméthyle, tous les R3 = H],
3-éthyle-3-(hydroxyméthyle)oxétane [c'est-à-dire, R1 = éthyle, R2 = hydroxyméthyle, tous les R3 = H] (= triméthylolpropanoxétane),
dérivés de triméthylolpropanoxétane éthoxylés et/ou propoxylés au groupe hydroxy et présentant 1 à 20 unités d'oxyde d'éthylène et/ou d'oxyde de propylène qui peuvent être agencées de façon statistique, en blocs ou de façon graduelle,
3,3-di(hydroxyméthyle)oxétane [c'est-à-dire, R1 = R2 = hydroxyméthyle, tous les R3 = H],
ou un mélange de ces hydroxyoxétanes,
de préférence 3-éthyle-3-(hydroxyméthyle)oxétane (= triméthylolpropanoxétane) et/ou au moins un dérivé de triméthylolpropanoxétane éthoxylé et/ou propoxylé au groupe hydroxy et présentant 1 à 20 unités d'oxyde d'éthylène et/ou d'oxyde de propylène qui peuvent etre agencées de façon statistique, en blocs ou de façon graduelle.

6. Procédé selon l'une quelconque des revendications précédentes, **caractérisé en ce que** le groupement de tête éthyléniquement non saturé U = acryloyle [H₂C = CH - C(=O)-] ou méthacryloyle [H₂C = C(CH₃) - C(=O)-].

7. Procédé selon l'une quelconque des revendications précédentes, **caractérisé en ce que** le groupement organique à deux liaisons présente la structure -O-L', dans laquelle -O- est un atome d'oxygène et L' est choisi dans le groupe consistant à groupements alkylène et groupements oxyde de polyalkylène.

8. Procédé selon l'une quelconque des revendications précédentes, **caractérisé en ce que** le groupe X - H est un groupe hydroxy O - H ou un groupe thiol S - H, de préférence un groupe hydroxy O - H.

9. Procédé selon l'une quelconque des revendications précédentes, **caractérisé en ce que** la molécule de départ U - L - X - H est choisie dans le groupe consistant à des esters d'hydroxyalkyle de l'acide méthacrylique [H₂C = C(CH₃) - C(=O) - O - (CH₂)ₐ - OH] et des esters de poly(oxyéthylène) de l'acide méthacrylique [H₂C = C(CH₃) - C(=O) - O - (CH₂ - CH₂ - O)ₐ- H], avec a = 1 à 20, de préférence a = 1 à 10 et plus préférablement a = 2 à 5.

10. Procédé selon l'une quelconque des revendications précédentes, **caractérisé en ce que** la molécule de départ U - L - X - H est du méthacrylate de hydroxyéthyle ou méthacrylate de hydroxybutyle.

11. Macromonomère qui peut être obtenu selon le procédé d'une quelconque des revendications 1 à 10.

12. Utilisation d'un macromonomère selon la revendication 11 pour la fabrication d'un polymère par polymérisation par radicaux.
